# NEW EUROPEAN PATENT SPECIFICATION

(11) **EP 1 294 899 B3**
(45) Date of publication of this specification: **06.07.2011**
(45) Mention of the grant of the patent: 10.06.2009
(21) Application number: 01922897.2
(22) Date of filing: 30.03.2001
(51) Int. Cl.: C12N 15/55, C12N 9/16, C12N 5/10, C07K 16/40, C12Q 1/68, G01N 33/50, G01N 33/577, G01N 33/68

(54) **16836, A HUMAN PHOSPHOLIPASE C FAMILY MEMBER AND USES THEREOF**
16836, EIN MITGLIED DER HUMANEN PHOSPHOLIPASE C FAMILIE UND SEINE VERWENDUNGEN
16836, UN NOUVEL ELEMENT DE LA FAMILLE DE LA PHOSPHOLIPASE C HUMAINE ET SES UTILISATIONS

(30) Priority: 31.03.2000 US 193921 P
(43) Date of publication of application: 26.03.2003
(73) Proprietor: MILLENNIUM PHARMACEUTICALS, INC., Cambridge, Massachusetts 02139 (US)
(72) Inventor: MEYERS, Rachel, A., Newton, MA 02468 (US); HUNTER, John, J., Somerville, MA 02143 (US)
(74) Representative: Taylor, Kate Laura
(86) International application number: PCT/US2001/010273
(87) International publication number: WO 2001/075121

(56) References cited:
- EP-A- 0 731 164
- EP-A- 1 074 617
- WO-A-00/58473
- WO-A-96/32485
- WO-A-98/45436
- WO-A-99/58675
- DATABASE EMBL SEQUENCE DATABASE [Online] Hinxton, UK; 2 January 2000 (2000-01-02) KAWASAKI ET AL.: "Homo sapiens pancreas-enriched phospholipase C mRNA, complete, cds." XP002180626
- DATABASE EMBL SEQUENCE DATABASE [Online] Hinxton, UK; 10 February 2000 (2000-02-10) ASHWELL: "Human DNA sequence from clone RP11-162K11 on chromosome 10" XP002180627
- DATABASE EMBL SEQUENCE DATABASE [Online] Hinxton, UK; 14 March 2000 (2000-03-14) REICHENSTEIN ET AL.: "Mus musculus phospholipase C-like protein mRNA, partial cds." XP002180628
- DATABASE EMBL SEQUENCE DATABSE [Online] Hinxton, UK; 23 May 2000 (2000-05-23) OHARA ET AL.: "Homo sapiens mRNA for KIAA1516 protein, partial cds." XP002180631 -& T. NAGASE ET AL.: "Prediction of the coding sequence of unidentified human genes XVII. The complete sequences of 100 new cDNA clones from brain which code for large proteins in vitro" DNA RESEARCH, vol. 7, April 2000 (2000-04), pages 143-150, XP000943428
- DATABASE EMBL SEQUENCE DATABASE [Online] Hinxton, UK; 2 October 2000 (2000-10-02) SONG ET AL.: "Homo sapiens phosphoinositide phospholipase C PLC-epsilon mRNA, complete cds." XP002180632
- DATABASE EMBL SEQUENCE DATABASE [Online] Hinxton, UK; 2 November 2000 (2000-11-02) LOPEZ ET AL.: "Homo sapiens phospholipase C epsilon mRNA partial cds." XP002180633

## Description

### Related Applications

This application claims priority to U.S. provisional application number 60/193,921 filed on March 31, 2000.

### Background of the Invention

Phosphoinositide-specific phospholipase C (PI-PLC) mediates the cellular actions of a variety of hormones, neurotransmitters, and growth factors. Activation of PI-PLC is one of the early responses to various extracellular signals. Agonist-dependent activation of PI-PLC causes hydrolysis of membrane phosphatidylinositol 4,5-bisphosphate (PIP₂), generating the second messengers inositol 1,4,5-trisphosphate (IP₃) and diacylglycerol (DAG). IP₃ binds specific intracellular receptors to trigger Ca²⁺ mobilization, while DAG mediates activation of a family of protein kinase C isozymes. This catalytic process is tightly regulated by reversible phosphorylation and binding of regulatory proteins (Rhee et al. (1997) J. Biol. Chem. 272:15045-15048).

In mammals, there are at least six different isoforms of PI-PLC, differing in their domain structure, regulation, and tissue distribution. Based on molecular size, immunoreactivity and amino acid sequence, several subtypes have been classified. Overall, sequence identity between sub-types is low, yet all isoforms share two conserved domains that constitute the PLC catalytic domain, designated X and Y: region X spans around 170 residues, and region Y about 260. The order of these two regions is always the same (NH2-X-Y-COOH), but the spacing is variable. In PLC-beta subtypes, X and Y domains are separated by a stretch of 70-120 amino acids rich in Ser, Thr and acidic residues, while their C-terminal 450 residues are rich in basic residues. In PLC-gammas, there is an insert of more than 400 residues containing one SH3 and two SH2 domains. PLCs show little similarity in the 300-residue N-terminal region preceding the X-domain. PI-PLCs have a C2 domain C-terminal of the catalytic domain. The C2 domain is thought to be involved in calcium-dependent phospholipid binding (Rhee et al. (1997) J. Biol. Chem. 272:15045-15048).

### Summary of the Invention

The present invention is based, in part, on the discovery of a novel phospholipase C family member, referred to herein as "16836". The nucleotide sequence of a cDNA encoding 16836 is shown in SEQ ID NO:1, and the amino acid sequence of a 16836 polypeptide is shown in SEQ ID NO:2. In addition, the nucleotide sequences of the coding region are depicted in SEQ ID NO:3.

Accordingly, in one aspect, the invention features a nucleic acid molecule that encodes a 16836 protein or polypeptide, e.g., a biologically active portion of the 16836 protein. In a preferred embodiment the isolated nucleic acid molecule encodes a polypeptide having the amino acid sequence of SEQ ID NO:2. In other embodiments, the invention provides isolated 16836 nucleic acid molecules having the nucleotide sequence shown in SEQ ID NO:1, SEQ ID NO:3, or the sequence of the DNA insert of the plasmid deposited with ATCC Accession Number 1774. In still other embodiments, the invention provides nucleic acid molecules that are substantially identical to the nucleotide sequence shown in SEQ ID NO:1, SEQ ID NO:3, or the sequence of the DNA insert of the plasmid deposited with ATCC Accession Number 1774. In other embodiments, the invention provides a nucleic acid molecule which hybridizes under a stringency condition described herein to a nucleic acid molecule comprising the nucleotide sequence of SEQ ID NO:1, SEQ ID NO:3, or the sequence of the DNA insert of the plasmid deposited with ATCC Accession Number 1774, wherein the nucleic acid encodes a full length 16836 protein or an active fragment thereof.

In a related aspect, the invention further provides nucleic acid constructs that include a 16836 nucleic acid molecule described herein. In certain embodiments, the nucleic acid molecules of the invention are operatively linked to native or heterologous regulatory sequences. Also included, are vectors and host cells containing the 16836 nucleic acid molecules of the invention e.g., vectors and host cells suitable for producing 16836 nucleic acid molecules and polypeptides.

In another related aspect, the invention provides nucleic acid fragments suitable as primers or hybridization probes for the detection of 16836-encoding nucleic acids.

In still another related aspect, isolated nucleic acid molecules that are antisense to a 16836 encoding nucleic acid molecule are provided.

In another aspect, the invention features, 16836 polypeptides, and biologically active or antigenic fragments thereof that are useful, e.g., as reagents or targets in assays applicable to treatment and diagnosis of 16836-mediated or -related disorders. In another embodiment, the invention provides 16836 polypeptides having a 16836 activity. Preferred polypeptides are 16836 proteins including at least one of: a Ras guanine nucleotide exchange factor (RasGEF) domain, a phosphatidylinositol-specific phospholipase C "X" domain, a phosphatidylinositol-specific phospholipase C "Y" domain, a C2 domain, or a Ras association (RaIGDS/AF-6) domain, and, preferably, having a 16836 activity, e.g., a 16836 activity as described herein.

In other embodiments, the invention provides 16836 polypeptides, e.g., a 16836 polypeptide having the amino acid sequence shown in SEQ ID NO:2 or the amino acid sequence encoded by the cDNA insert of the plasmid deposited with ATCC Accession Number 1774; an amino acid sequence that is substantially identical to the amino acid sequence shown in SEQ ID NO:2 or the amino acid sequence encoded by the cDNA insert of the plasmid deposited with ATCC Accession Number 1774; or an amino acid sequence encoded by a nucleic acid molecule having a nucleotide sequence which hybridizes under a stringency condition described herein to a nucleic acid molecule comprising the nucleotide sequence of SEQ ID NO:1, SEQ ID NO:3, or the sequence of the DNA insert of the plasmid deposited with ATCC Accession Number 1774, wherein the nucleic acid encodes a full length 16836 protein or an active fragment thereof.

In a related aspect, the invention further provides nucleic acid constructs which include a 16836 nucleic acid molecule described herein.

In a related aspect, the invention provides 16836 polypeptides or fragments operatively linked to non-16836 polypeptides to form fusion proteins.

In another aspect, the invention features antibodies and antigen-binding fragments thereof, that react with, or more preferably specifically bind 16836 polypeptides.

In another aspect, the invention provides methods of screening for agents, e.g., compounds, that modulate the expression or activity of the 16836 polypeptides or nucleic acids.

In still another aspect, the invention provides a process for modulating 16836 polypeptide or nucleic acid expression or activity, e.g. using the screened compounds. Also include in the disclosure, the methods involve treatment of conditions related to aberrant activity or expression of the 16836 polypeptides or nucleic acids, such as conditions involving aberrant or deficient cellular proliferation or differentiation.

In yet another aspect, the invention provides methods of modulating the activity of a 16836-expressing cell, e.g., a 16836-expressing hyperproliferative or aberrant cell. The method includes contacting the cell with a compound (e.g., a compound identified using the methods described herein) that modulates the activity, or expression, of the 16836 polypeptide or nucleic acid, thereby modulating the cell activity. Preferably, the activity of the cell is modulated by inhibiting the proliferation, or inducing the differentiation or killing, of the 16836-expressing cell.

In a preferred embodiment, the contacting step is effective *in vitro* or *ex vivo.* In other embodiments, the contacting step is effected *in vivo,* e.g., in a subject (e.g., a mammal, e.g., a human), as part of a therapeutic or prophylactic protocol.

In a preferred embodiment, the 16836-expressing cell is found in a cancer tissue or cell, e.g., a solid tumor, a soft tissue tumor, or a metastatic lesion. In one example, the 16836-expressing cell is found in a cancerous tissue from the lung, colon, or breast.

In other embodiments, the 16836-expressing cell is a bone cell (e.g., an osteoclast), a brain cell, a testicular cell, a heart cell, a skeletal muscle cell, or an immune cell, e.g., a cell from a myeloid, lymphoid or erythroid lineage, or a precursor cell thereof.

In a preferred embodiment, the agent, e.g., compound, is an inhibitor of a 16836 polypeptide. Preferably, the inhibitor is chosen from a peptide, a phosphopeptide, a peptidomimetic, e.g., a phosphonate analog of a peptide substrate, a small organic molecule, a small inorganic molecule and an antibody (e.g., an antibody conjugated to a therapeutic moiety selected from a cytotoxin, a cytotoxic agent and a radioactive metal ion).

In a preferred embodiment, the agent, e.g., compound, is an inhibitor of a 16836 nucleic acid, e.g., an antisense, a ribozyme, or a triple helix molecule.

In a preferred embodiment, the agent, e.g., compound, is administered in combination with a cytotoxic agent. Examples of cytotoxic agents include anti-microtubule agent, a topoisomerase I inhibitor, a topoisomerase II inhibitor, an antimetabolite, a mitotic inhibitor, an alkylating agent, an intercalating agent, an agent capable of interfering with a signal transduction pathway, an agent that promotes apoptosis or necrosis, and radiation.

In another aspect, the invention features a method of treating, or preventing, in a subject, a disorder characterized by aberrant activity or expression of a 16836 nucleic acid or polypeptide. In one embodiment, the method includes administering to the subject an effective amount of an agent, e.g., a compound, that modulates the activity or expression of a 16836 polypeptide or nucleic acid such that the disorder is ameliorated or prevented.

In a preferred embodiment, the disorder is a cellular proliferative or differentiative disorder, e.g., lung cancer, colon cancer, or breast cancer.

In a preferred embodiment, the subject is a human. For example, the subject is a human suffering from, or at risk of, a cellular proliferative and/or differentiative disorder as described herein. In other embodiments, the subject is a human suffering from, or at risk of, a bone disorder, a brain disorder, a cardiovascular disorder, a reproductive disorder, a skeletal muscle disorder, or an immune disorder as described herein.

In a preferred embodiment, the agent, e.g., compound, is a peptide, a phosphopeptide, a small molecule, an antibody, or any combination thereof. In another embodiment, the agent is an antisense, a ribozyme, a triple helix molecule, a 16836 nucleic acid, or any combination thereof.

In a preferred embodiment, the agent, e.g., compound, is administered in combination with a cytotoxic agent. Examples of cytotoxic agents include anti-microtubule agent, a topoisomerase I inhibitor, a topoisomerase II inhibitor, an antimetabolite, a mitotic inhibitor, an alkylating agent, an intercalating agent, an agent capable of interfering with a signal transduction pathway, an agent that promotes apoptosis or necrosis, and radiation.

The invention also provides assays for determining the activity of or the presence or absence of 16836 polypeptides or nucleic acid molecules in a biological sample, including for disease diagnosis. Preferably, the biological sample includes a cancerous or pre-cancerous cell or tissue. For example, the cancerous tissue can be a solid tumor, a soft tissue tumor, or a metastatic lesion. In one example, the cancerous tissue is from the lung, colon, or breast. In other embodiments, the biological sample contains a bone cell (e.g., an osteoclast), a brain cell, a testicular cell, a heart cell, a skeletal muscle cell, or an immune cell, e.g., a cell from a myeloid, lymphoid or erythroid lineage, or a precursor cell thereof.

In further aspect, the invention provides assays for determining the presence or absence of a genetic alteration in a 16836 polypeptide or nucleic acid molecule, including for disease diagnosis. Preferably, the biological sample includes a cancerous or pre-cancerous cell or tissue. For example, the cancerous tissue can be a solid tumor, a soft tissue tumor, or a metastatic lesion. In one example, the cancerous tissue is from the lung, colon, or breast. In other embodiments, the biological sample contains a bone cell (e.g., an osteoclast), a brain cell, a testicular cell, a skeletal muscle cell, or an immune cell, e.g., a cell from a myeloid, lymphoid or erythroid lineage, or a precursor cell thereof.

In another aspect, the invention features a method of diagnosing, or staging, a 16836-mediated disorder, e.g., a cellular proliferative and/or differentiative disorder, in a subject. The method includes evaluating the expression or activity of a 16836 nucleic acid or polypeptide, thereby diagnosis or staging the disorder. In a preferred embodiment, the expression or activity is compared with a reference value, e.g., a difference in the expression or activity level of the 16836 nucleic or polypeptide relative to a normal subject or a cohort of normal subjects is indicative of the disorder, or a stage in the disorder.

In a preferred embodiment, the subject is a human. For example, the subject is a human suffering from, or at risk of, a cellular proliferative and/or differentiative disorder as described herein.

In a preferred embodiment, the evaluating step occurs *in vitro* or ex vivo. For example, a sample, e.g., a blood or tissue sample, a biopsy, is obtained from the subject. Preferably, the sample contains a cancer cell.

Also included in the disclosure, the evaluating step occurs *in vivo.* For example, by administering to the subject a detectably labeled agent that interacts with the 16836-associated nucleic acid or polypeptide, such that a signal is generated relative to the level of activity or expression of the 16836 nucleic acid or polypeptide.

In preferred embodiments, the method is performed: on a sample from a subject, a sample from a human subject; e.g., a sample of a patient suffering from, or at risk of, a cellular proliferative and/or differentiative disorder as described herein; to determine if the individual from which the target nucleic acid or protein is taken should receive a drug or other treatment; to diagnose an individual for a disorder or for predisposition to resistance to treatment, to stage a disease or disorder.

In a still further aspect, the invention provides methods for evaluating the efficacy of a treatment of a disorder, e.g., a cellular proliferative and/or differentiative, e.g., a cancer. The method includes: treating a subject, e.g., a patient or an animal, with a protocol under evaluation (e.g., treating a subject with one or more of: chemotherapy, radiation, and/or a compound identified using the methods described herein); and evaluating the expression of a 16836 nucleic acid or polypeptide before and after treatment. A change, e.g., a decrease or increase, in the level of a 16836 nucleic acid (e.g., mRNA) or polypeptide after treatment, relative to the level of expression before treatment, is indicative of the efficacy of the treatment of the disorder.

In a preferred embodiment, the evaluating step includes obtaining a sample (e.g., a tissue sample, e.g., a biopsy, or a fluid sample) from the subject, before and after treatment and comparing the level of expressing of a 16836 nucleic acid (e.g., mRNA) or polypeptide before and after treatment.

In another aspect, the invention provides methods for evaluating the efficacy of a therapeutic or prophylactic agent (e.g., an anti-neoplastic agent). The method includes: contacting a sample with an agent (e.g., a compound identified using the methods described herein, a cytotoxic agent) and, evaluating the expression or activity of a 16836 nucleic acid or polypeptide in the sample before and after the contacting step. A change, e.g., a decrease or increase, in the level of 16836 nucleic acid (e.g., mRNA) or polypeptide in the sample obtained after the contacting step, relative to the level of expression in the sample before the contacting step, is indicative of the efficacy of the agent. The level of 16836 nucleic acid or polypeptide expression can be detected by any method described herein.

In a preferred embodiment, the sample includes cells obtained from a cancerous tissue where a 16836 polypeptide or nucleic acid is obtained.

In a preferred embodiment, the sample is a tissue sample (e.g., a biopsy), a bodily fluid, a cultured cell (e.g., a tumor cell line).

In other embodiments, the sample includes a bone cell (e.g., an osteoclast), a testicular cell, a heart cell, a skeletal muscle cell, or an immune cell, e.g., a cell from a myeloid, lymphoid or erythroid lineage, or a precursor cell thereof.

In another aspect, the invention features a method for identifying an agent that modulates the activity or expression of a 16836 polypeptide or nucleic acid. The method includes the steps of: contacting the 16836 polypeptide or nucleic acid with an agent; and determining the effect of the agent on the activity or expression of the polypeptide or nucleic acid. In one embodiment, the method includes contacting a 16836 polypeptide with the agent and determining the effect of the agent on the ability of the 16836 polypeptide to catalyze the hydrolysis of phosphatidylinositol. In another embodiment, the method includes contacting a 16836 polypeptide with the agent and determining the effect of the agent on the ability of the 16836 polypeptide to associate with a Ras protein. In another embodiment, the method includes contacting a 16836 polypeptide with the agent and determining the effect of the agent on the ability of the 16836 polypeptide to mediate guanine nucleotide exchange activity. The agent can be a peptide, a phosphopeptide, a small molecule, an antibody, or any combination thereof. In addition, the agent can be an antisense, a ribozyme, a triple helix molecule, a 16836 nucleic acid, or any combination thereof.

Other features and advantages of the invention will be apparent from the following detailed description, and from the claims.

### Brief Description of the Drawings

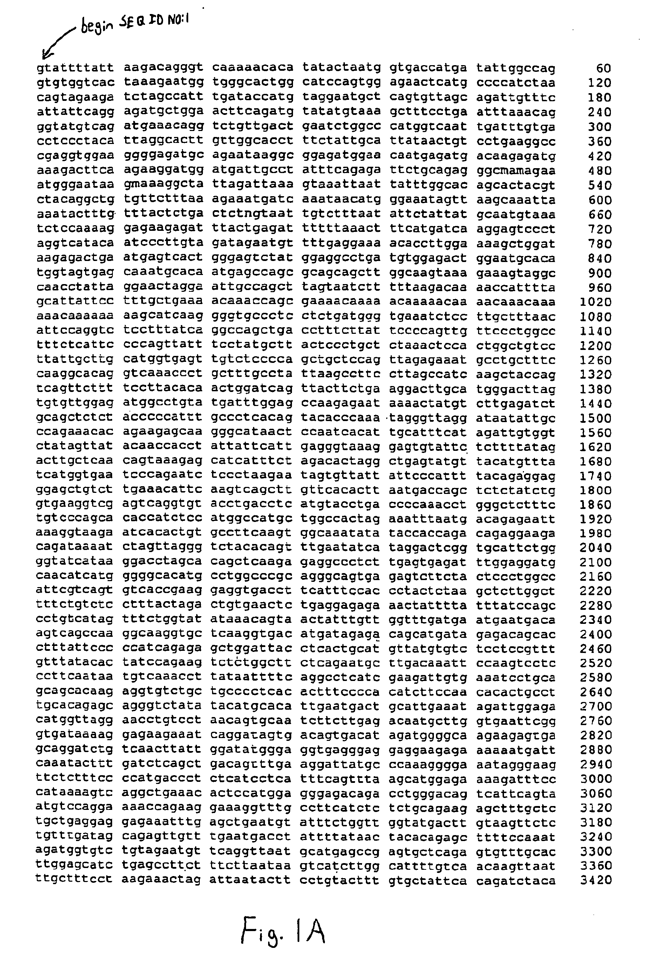

*Figures 1A-1J* depict a cDNA sequence (SEQ ID NO:1) and predicted amino acid sequence (SEQ ID NO:2) of human 16836. The methionine-initiated open reading frame of human 16836 (without the 5' and 3' untranslated regions) is shown as coding sequence SEQ ID NO:3.

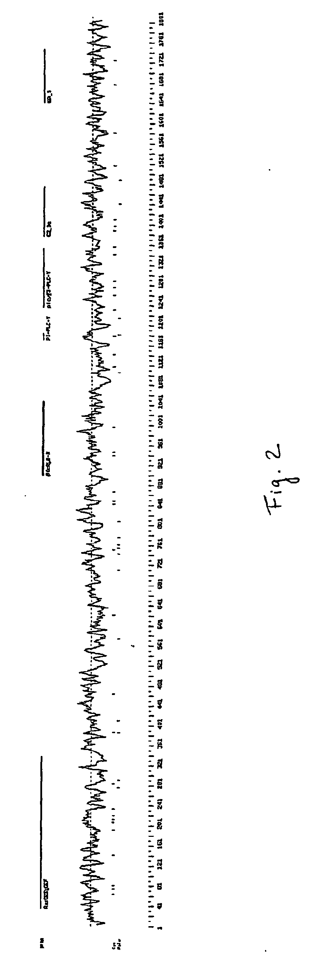

*Figure 2* depicts a hydropathy plot of human 16836. Relative hydrophobic residues are shown above the dashed horizontal line, and relative hydrophilic residues are below the dashed horizontal line. The cysteine residues (cys) are indicated by short vertical lines just below the hydropathy trace. The numbers corresponding to the amino acid sequence of human 16836 are indicated. Polypeptides of the invention include fragments which include: all or part of a hydrophobic sequence, i.e., a sequence above the dashed line, e.g., the sequence from about amino acid 200-230, from about 430-450, and from about 830-840 of SEQ ID NO:2; all or part of a hydrophilic sequence, i.e., a sequence below the dashed line, e.g., the sequence of from about amino acid 330-350, from about 610-630, and from about 1120-1140 of SEQ ID NO:2; a sequence which includes a Cys, or a glycosylation site.

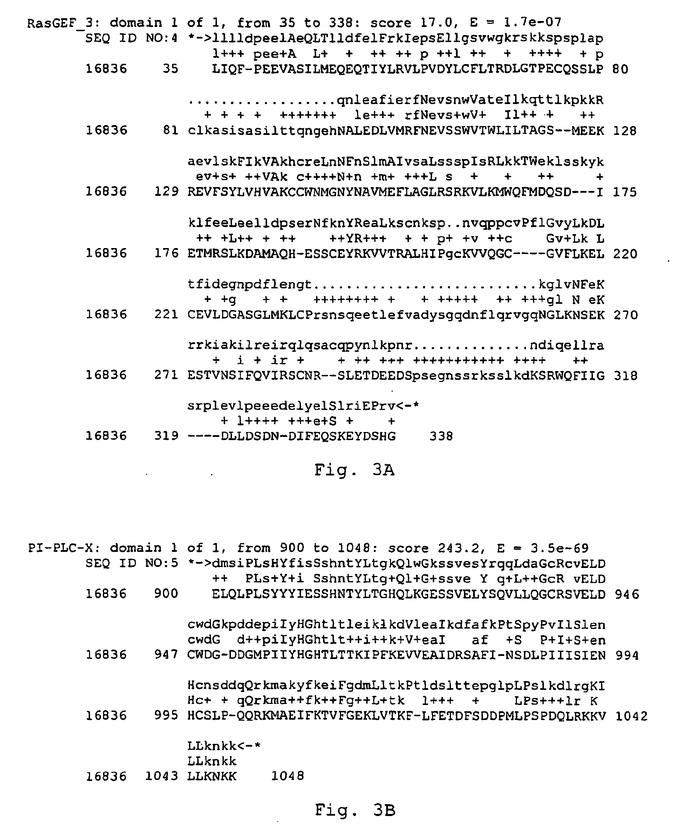

*Figure 3A* depicts an alignment of the Ras guanine nucleotide exchange factor (RasGEF) domain of human 16836 with a consensus amino acid sequence derived from a hidden Markov model (HMM) from SMART. The upper sequence is the consensus amino acid sequence (SEQ ID NO:4), while the lower amino acid sequence corresponds to amino acids 35-338 of SEQ ID NO:2.
*Figure 3B* depicts an alignment of the PI-PLC-X domain of human 16836 with a consensus amino acid sequence derived from a hidden Markov model (HMM) from PFAM. The upper sequence is the consensus amino acid sequence (SEQ ID NO:5), while the lower amino acid sequence corresponds to amino acids 900-1048 of SEQ ID NO:2.

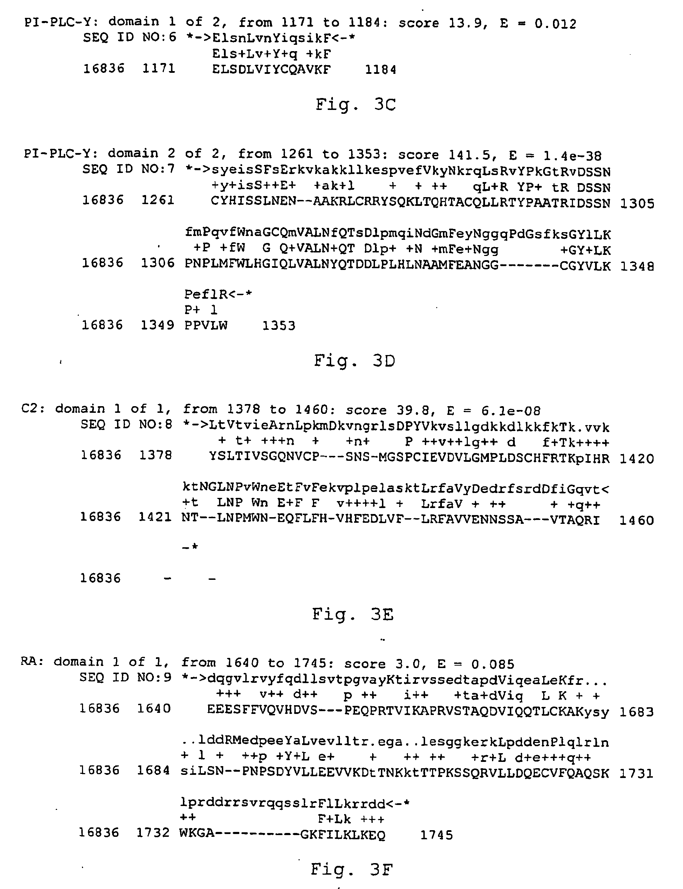

*Figure 3C* depicts an alignment of a PI-PLC-Y domain of human 16836 with a consensus amino acid sequence derived from a hidden Markov model (HMM) from PFAM. The upper sequence is the consensus amino acid sequence (SEQ ID NO:6), while the lower amino acid sequence corresponds to amino acids 1171-1184 of SEQ ID NO:2.
*Figure 3D* depicts an alignment of a PI-PLC-Y domain of human 16836 with a consensus amino acid sequence derived from a hidden Markov model (HMM) from PFAM. The upper sequence is the consensus amino acid sequence (SEQ ID NO:7), while the lower amino acid sequence corresponds to amino acids 1261-1353 of SEQ ID NO:2.
*Figure 3E* depicts an alignment of the C2 domain of human 16836 with a consensus amino acid sequence derived from a hidden Markov model (HMM) from PFAM. The upper sequence is the consensus amino acid sequence (SEQ ID NO:8), while the lower amino acid sequence corresponds to amino acids 1378-1460 of SEQ ID NO:2.
*Figure 3F* depicts an alignment of the Ras association (RaIGDS/AF-6) domain of human 16836 with a consensus amino acid sequence derived from a hidden Markov model (HMM) from PFAM. The upper sequence is the consensus amino acid sequence (SEQ ID NO:9), while the lower amino acid sequence corresponds to amino acids 1640-1745 of SEQ ID NO:2.

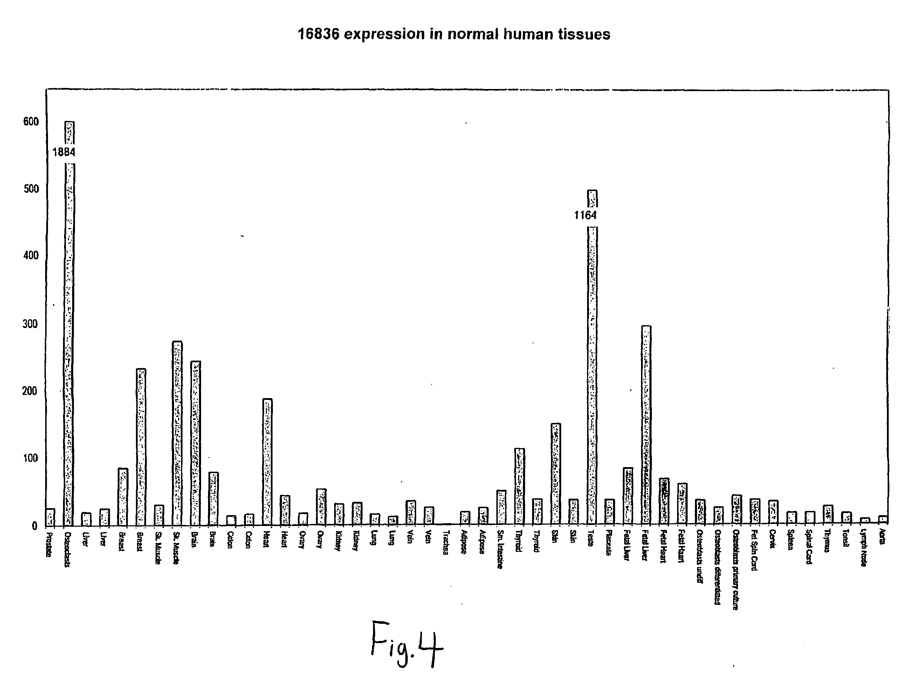

*Figure 4* is a bar graph depicting the expression of 16836 RNA in a panel of normal human tissues, including bone cells, fetal liver, bone marrow, trachea, skin, skeletal muscle, testis, detected using TaqMan analysis.

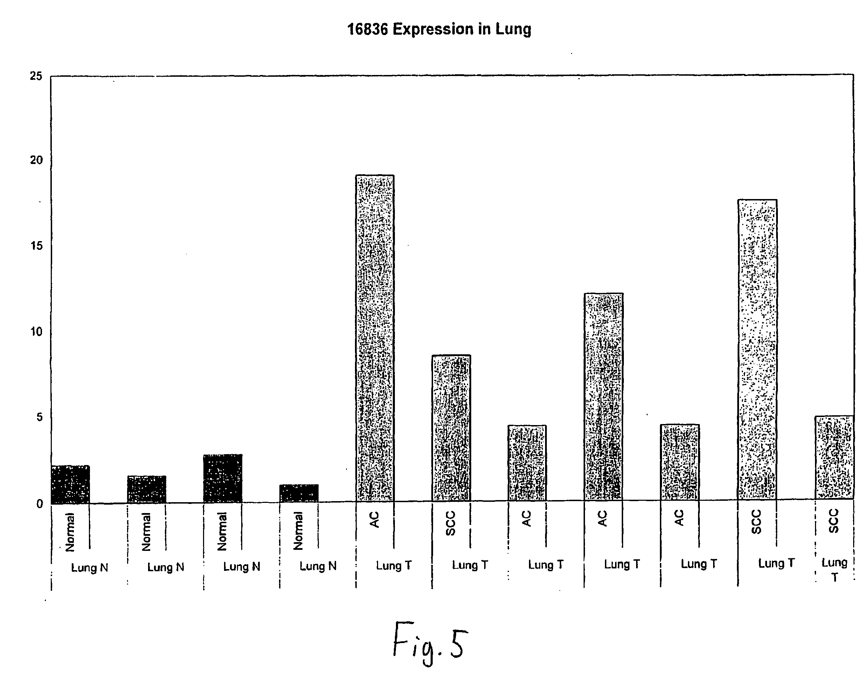

*Figure 5* is a bar graph depicting the expression of 16836 RNA in a panel of normal lung cells and lung tumor cells, relative to normal cells. Expression was detected using TaqMan analysis.

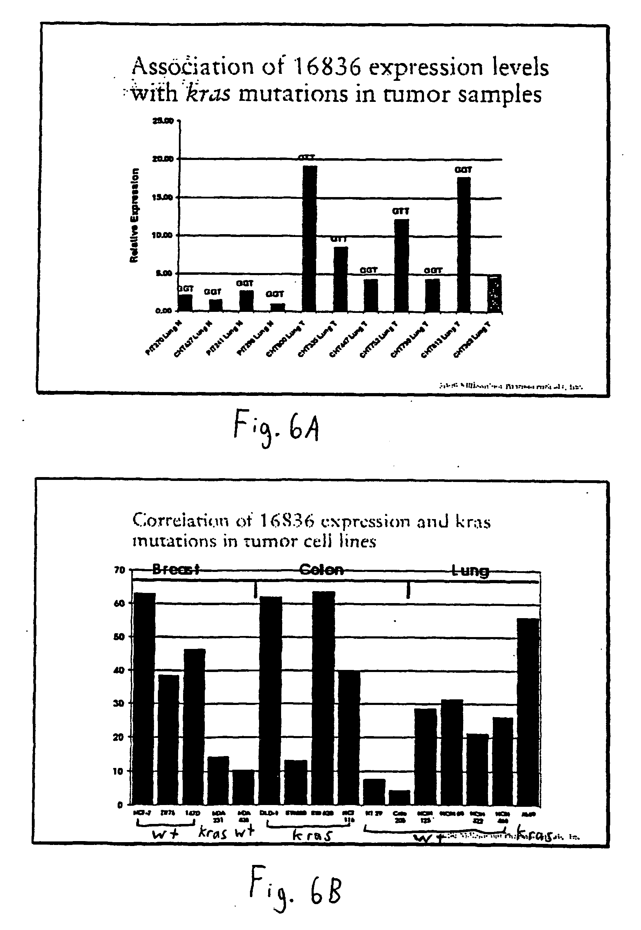

*Figure 6A* is a bar graph depicting the association of 16836 expression levels with kras mutations in tumor (T) and normal (N) samples. Columns indicated as "GTT" correspond to kras mutant samples, whereas columns indicated "GGT" correspond to wild type samples. Expression was detected using TaqMan analysis.
*Figure 6B* is a bar graph depicting the correlation of 16836 expression and kras mutations in tumor cell lines. Columns indicated as "kras" correspond to kras mutant samples; columns indicated as "wt" correspond to wild type samples. Expression was detected using TaqMan analysis.

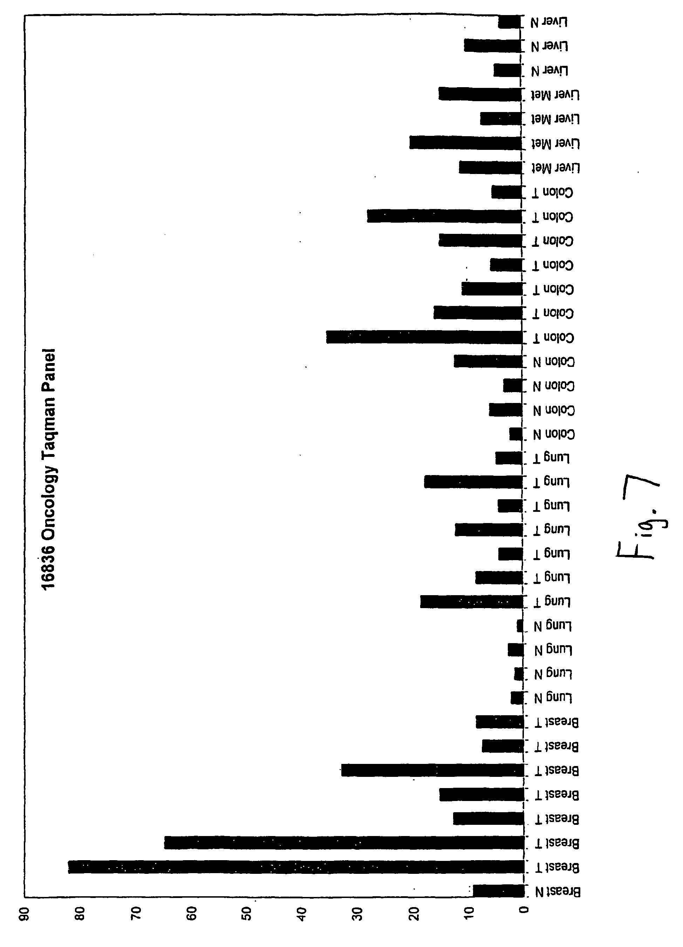

*Figure* 7 is a bar graph depicting 16836 expression in normal and malignant breast, lung, colon and liver tissues.

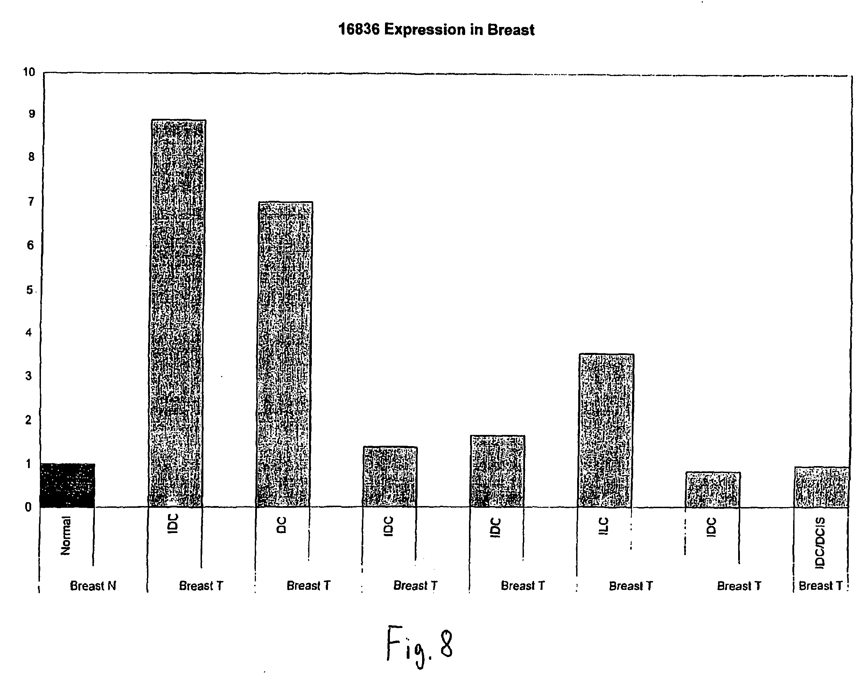

*Figure 8* is a bar graph depicting 16836 expression in normal and malignant breast tissue samples, including infiltrating ductal carcinoma (IDC), ductal carcinoma (DC), invasive lobular carcinoma (ILC), and ductal carcinoma in situ (DCIS).

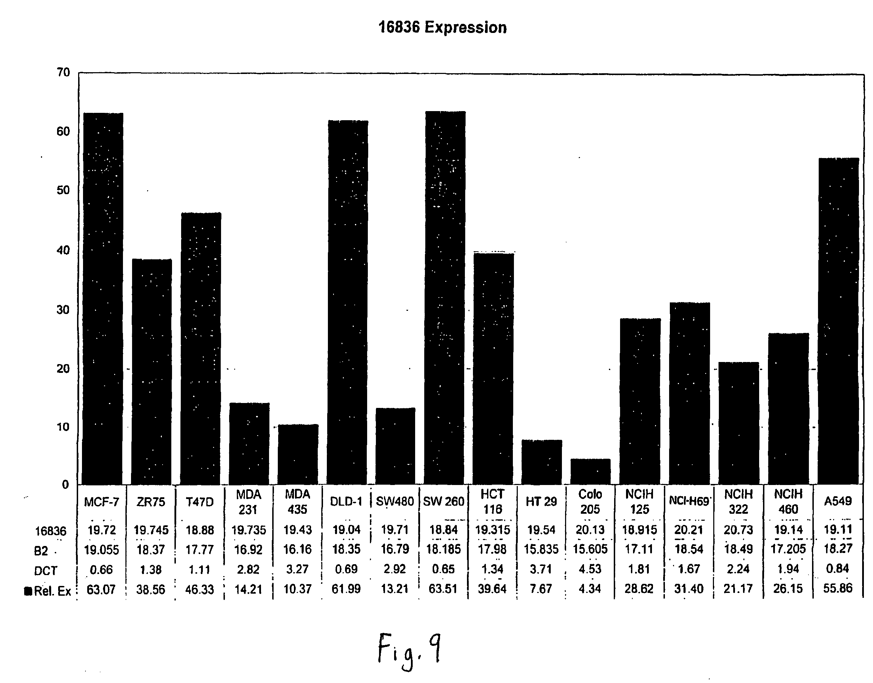

*Figure 9* is a bar graph depicting 16836 expression in cancer cell lines, including human breast cancer cell lines, human colon cancer cell lines, and human lung cancer cell lines.

### Detailed Description

The human 16836 sequence (Figs. 1A-1J; SEQ ID NO:1), which is approximately 10,172 nucleotides long including untranslated regions, contains a predicted coding sequence of about 5,430 nucleotides, including the termination codon (nucleotides indicated as coding of SEQ ID NO:1 in Figs. 1A-1J; SEQ ID NO:3). The coding sequence encodes a 1809 amino acid protein (SEQ ID NO:2).

Human 16836 contains the following regions or structural features: a Ras guanine nucleotide exchange factor (RasGEF) domain (Fig. 3A; SMART identifier RasGEF) located at about amino acid residues 35-338 of SEQ ID NO:2; a phosphatidylinositol-specific phospholipase C "X" (PI-PLC-X) domain (Fig. 3B; PFAM Accession PF00388) located at about amino acid residues 900-1048 of SEQ ID NO:2; a phosphatidylinositol-specific phospholipase C "Y" (PI-PLC-Y) domain (Figs. 3C and 3D; PFAM Accession PF00387) located at about amino acid residues 1171-1184 and 1261-1353 of SEQ ID NO:2; a C2 domain (Fig. 3E; PFAM Accession PF00168) located at about amino acid residues 1378-1460 of SEQ ID NO:2; and a Ras association (RaIGDS/AF-6) (RA) domain (Fig. 3F; PFAM Accession PF00788) located at about amino acid residues 1640-1745 of SEQ ID NO:2.

The 16836 protein also includes the following domains: 16 predicted N-glycosylation sites (PS00001) located at about amino acids 285-288, 300-303, 395-398, 419-422, 583-586, 719-722, 752-755, 764-767, 770-773, 784-787, 817-820, 1115-1118, 1191-1194, 1224-1227, 1450-1453, and 1498-1501 of SEQ ID NO:2; six predicted cAMP- and cGMP-dependent protein kinase phosphorylation sites (PS00004) located at about amino acids 303-306, 559-562, 1162-1165, 1277-1280, 1707-1710, and 1764-1767 of SEQ ID NO:2; 30 predicted protein kinase C phosphorylation sites (PS00005) located at about amino acids 160-162, 177-179, 180-182, 268-270, 301-303, 306-308, 342-344, 384-386, 410-412, 501-503, 562-564, 571-573, 590-592, 613-615, 653-655, 686-688, 733-735, 736-738, 964-966, 1195-1197, 1203-1205, 1227-1229, 1258-1260, 1280-1282, 1492-1494, 1520-1522, 1705-1707, 1711-1713, 1715-1717, and 1769-1771 of SEQ ID NO:2; 37 predicted casein kinase II phosphorylation sites (PS00006) located at about amino acids 124-127, 173-176, 180-183, 190-193, 239-242, 251-254, 268-271, 290-293, 295-298, 306-309, 323-326, 437-440, 607-610, 626-629, 721-724, 736-739, 754-757, 766-769, 819-822, 849-852, 889-892, 927-930, 1054-1057, 1102-1105, 1117-1120, 1216-1219, 1230-1233, 1238-1241, 1266-1269, 1299-1302, 1364-1367, 1618-1621, 1638-1641, 1667-1670, 1749-1752, 1790-1793, and 1800-1803 of SEQ ID NO:2; 16 predicted N-myristoylation sites (PS00008) located at about amino acids 71-76, 146-151, 264-269, 338-343, 416-421, 484-489, 587-592, 705-710, 820-825, 1194-1199, 1209-1214, 1234-1239, 1501-1506, 1541-1546, 1756-1761, and 1797-1802 of SEQ ID NO:2; one predicted amidation site (PS00009) located at about amino acids 557-560 of SEQ ID NO:2; and one predicted coiled coil domain at about amino acids 874-901 of SEQ ID NO:2.

For general information regarding PFAM identifiers, PS prefix and PF prefix domain identification numbers, refer to Sonnhammer et al. (1997) Protein 28:405-420 and http://www.psc.edu/general/ software/packages/pfam/pfam.html.

A plasmid containing the nucleotide sequence encoding human 16836 (clone "Fbh16836FL") was deposited with American Type Culture Collection (ATCC), 10801

University Boulevard, Manassas, VA 20110-2209, on April 19, 2000 and assigned Accession Number 1774. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

**Table 1: Summary of Sequence Information for 16836**

| **cDNA** | **ORF** | **Polypeptide** | **Figure** | **ATCC Accession Number** |
|---|---|---|---|---|
| SEQ ID NO:1 | SEQ ID NO:3 | SEQ ID NO:2 | Figs. 1A-1J | 1774 |

**Table 2: Summary of Domains of 16836**

| **Domain** | **Location in SEQ ID NO:2** |
|---|---|
| RasGEF | about amino acid residues 35-338 of SEQ ID NO:2 |
| PI-PLC-X | about amino acid residues 900-1048 of SEQ ID NO:2 |
| PI-PLC-Y | about amino acid residues 1171-1184 and 1261-1353 of SEQ ID NO:2 |
| C2 | about amino acid residues 1378-1460 of SEQ ID NO:2 |
| Ras association (RA) | about amino acid residues 1640-1745 of SEQ ID NO:2 |

The 16836 protein contains a significant number of structural characteristics in common with members of the phospholipase C family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., rat or mouse proteins. Members of a family can also have common functional characteristics.

Members of the phosphoinositide -specific phospholipase C (PI-PLC) family of proteins are characterized by an amino acid sequence that catalyzes the hydrolysis of phosphatidyl-inositol-4,5-bisphosphate (PIP₂) to generate the second messengers inositol 1,4,5-trisphosphate (IP₃) and diacylglycerol (DAG). IP₃ can diffuse into the endoplasmic reticulum surface where it can bind an IP₃ receptor, inducing the release of Ca²⁺ from intracellular stores into the cytoplasm. DAG remains in the cell membrane where it can serve to activate the enzyme protein kinase C. Both Ca²⁺ release and protein kinase C activation are involved in cellular events such as proliferation, differentiation, secretion, and migration.

Members of the PI-PLC family generally share one or more of the following domains: a PI-PLC-X domain; a PI-PLC-Y domain; and a C2 domain. A PI-PLC-X domain is a subdomain of PI-PLC that, together with a PI-PLC-Y subdomain, constitutes the catalytic site of a phospholipase, e.g., a domain that catalyzes the hydrolysis of phosphatidylinositol. A C2 domain is a domain that can mediate calcium dependent binding to phospholipids.

The domain structure of 16836 is similar to the domain structure of a protein encoded by the *C. elegans* gene, PLC210 (Shibatohge et al. (1998) J. Biol. Chem. 273:6218-6222). PLC210 was isolated in a yeast two-hybrid screen for effectors of ras function. PLC210 binds preferentially to GTP-bound ras (active), suggesting that it functions in propagating or amplifying signals for cellular proliferation. In addition to a role for 16836 and PLC210 as ras effectors, their membership in the PI-PLC family also suggests a functional role for the protein in proliferation signaling.

16836 and PLC210 appear to contain domains not found in members of the three known classes ofPI-PLCs. First, the N terminal region harbors a domain, a Ras guanine nucleotide exchange factor (RasGEF) domain, homologous to a family of guanine nucleotide exchange factors for ras. Second, the C terminal region contains a structure for ras binding, a ras association (RA) domain. A RA domain is a domain that can mediate binding to a ras protein. The RA domain associates preferentially with an activated ras, e.g. the RA domain may specifically associate with GTP-bound ras. Furthermore, the RA domain interacts with the effector region of ras. Thus, 16836 and PLC210 comprise a novel class of PI-PLC.

Ras may regulate the activity of 16836 by modulating one or more of: (1) an activation of 16836 activity; or (2) a ras-induced translocation of 16836 to a specific membrane compartment containing substrates upon which 16836 may act. The increased rate of phosphoinositide turnover observed in ras-transformed cells suggests a persistent PI-PLC stimulation by activated ras. Furthermore, anti-PI-PLC antibodies inhibit ras-induced mitogenesis. Thus, the regulation of PI-PLC by ras may play a role in controlling cell proliferation and/or differentiation.

A 16836 molecule can include a "Ras guanine nucleotide exchange factor (RasGEF)" domain or regions homologous with a "RasGEF" domain.

As used herein, the term "RasGEF domain" includes an amino acid sequence of about 50-400 amino acid residues in length and having a bit score for the alignment of the sequence to the RasGEF domain profile (SMART HMM) of at least 5. Preferably, a RasGEF domain includes at least about 80-350 amino acids, more preferably about 150-325 amino acid residues, or about 250-320 amino acids and has a bit score for the alignment of the sequence to the RasGEF domain (HMM) of at least 15 or greater. The RasGEF domain (HMM) has been assigned the SMART identifier RasGEF (http://smart.embl-heidelber.de/). The RasGEF domain (HMM) has been assigned the PFAM Accession Number PF00617 (http;//genome.wustl.edu/Pfam/.html). An alignment of the RasGEF domain (amino acids 35-338 of SEQ ID NO:2) of human 16836 with a consensus amino acid sequence (SEQ ID NO:4) derived from a hidden Markov model is depicted in Figure 3A.

In a preferred embodiment 16836 polypeptide or protein of the disclosure has a "RasGEF domain" or a region which includes at least about 80-350 more preferably about 150-325 or 250-320 amino acid residues and has at least about 50%, 60%, 70% 80% 90% 95%, 99%, or 100% homology with a "RasGEF domain," e.g., a RasGEF domain of human 16836 (e.g., residues 35-338 of SEQ ID NO:2).

A 16836 polypeptide of the disclosure can further include a "PI-PLC-X domain" or regions homologous with a "PI-PLC-X domain."

As used herein, the term "PI-PLC-X domain" includes an amino acid sequence of about 30-250 amino acid residues in length and having a bit score for the alignment of the sequence to the PI-PLC-X domain profile (PFAM HMM) of at least 50. Preferably, a PI-PLC-X domain includes at least about 100-220 amino acids, more preferably about 120-200 amino acid residues, or about 130-170 amino acids and has a bit score for the alignment of the sequence to the PI-PLC-X domain (HMM) of at least 240 or greater.

The PI-PLC-X domain (HMM) has been assigned the PFAM Accession Number PF00388 (http;//genome.wustl.edu/Pfam/html). An alignment of the PI-PLC-X domain (amino acids 900-1048 of SEQ ID NO:2) of human 16836 with a consensus amino acid sequence (SEQ ID NO:5) derived from a hidden Markov model is depicted in Figure **3B****.**

In a preferred embodiment 16836 polypeptide or protein of the disclosure has a "PI-PLC-X domain" or a region which includes at least about 100-220 more preferably about 120-200 or 130-170 amino acid residues and has at least about 50%, 60%, 70% 80% 90% 95%, 99%, or 100% homology with a "PI-PLC-X domain," e.g., the PI-PLC-X domain of human 16836 (e.g., residues 900-1048 of SEQ ID NO:2).

A 16836 molecule can of the disclosure further include a "PI-PLC-Y domain" or regions homologous with a "PI-PLC-Y domain."

As used herein, the term "PI-PLC-Y domain" includes an amino acid sequence of about 40-300 amino acid residues in length and having a bit score for the alignment of the sequence to the PI-PLC-Y domain profile (PFAM HMM) of at least 10. Preferably, a PI-PLC-Y domain includes at least about 60-260 amino acids, more preferably about 80-250 amino acid residues, or about 90-200 amino acids and has a bit score for the alignment of the sequence to the PI-PLC-X domain (HMM) of at least 140 or greater. The PI-PLC-Y domain (HMM) has been assigned the PFAM Accession Number PF00387 (http;//genome.wustl.edu/Pfam/.html). Alignments of the PI-PLC-Y domains (amino acids 1171-1184 and 1261-1353 of SEQ ID NO:2) of human 16836 with consensus amino acid sequences (SEQ ID NO:6 and SEQ ID NO:7) derived from a hidden Markov model are depicted in Figures 3C and 3D.

In a preferred embodiment 16836 polypeptide or protein of the disclosure has a "PI-PLC-Y domain" or a region which includes at least about 60-260 more preferably about 80-250 or 90-200 amino acid residues and has at least about 50%, 60%, 70% 80% 90% 95%, 99%, or 100% homology with a "PI-PLC-Y domain," e.g., a PI-PLC-Y domain of human 16836 (e.g., residues 1171-1184 and 1261-1353 of SEQ ID NO:2).

A 16836 molecule of the disclosure can further include a "C2 domain" or regions homologous with a "C2 domain."

As used herein, the term "C2 domain" includes an amino acid sequence of about 20-200 amino acid residues in length and having a bit score for the alignment of the sequence to the C2 domain profile (PFAM HMM) of at least 15. Preferably, a C2 domain includes at least about 50-120 amino acids, more preferably about 70-100 amino acid residues, or about 80-90 amino acids and has a bit score for the alignment of the sequence to the C2 domain (HMM) of at least 35 or greater. The C2 domain (HMM) has been assigned the PFAM Accession Number PF00168 (http;//genome.wustl.edu/Pfam/.html). An alignment of the C2 domain (amino acids 1378-1460 of SEQ ID NO:2) of human 16836 with a consensus amino acid sequence (SEQ ID NO:8) derived from a hidden Markov model is depicted in Figure 3E.

In a preferred embodiment 16836 polypeptide or protein of the disclosure has a "C2 domain" or a region which includes at least about 50-120 more preferably about 70-100 or 80-90 amino acid residues and has at least about 50%, 60%, 70% 80% 90% 95%, 99%, or 100% homology with a "C2 domain," e.g., a C2 domain of human 16836 (e.g., residues 1378-1460 of SEQ ID NO:2).

A 16836 molecule of the disclosure can further include a "Ras association (RA) domain" or regions homologous with a " RA domain."

As used herein, the term "RA domain" includes an amino acid sequence of about 20-200 amino acid residues in length and having a bit score for the alignment of the sequence to the RA domain profile (PFAM HMM) of at least 2. Preferably, a RA domain includes at least about 50-140 amino acids, more preferably about 80-120 amino acid residues, or about 90-110 amino acids and has a bit score for the alignment of the sequence to the RA domain (HMM) of at least 3 or greater. The RA domain (HMM) has been assigned the PFAM Accession Number PF00788 (http;//genome.wustl.edu/Pfam/.html). An alignment of the RA domain (amino acids 1640-1745 of SEQ ID NO:2) of human 16836 with a consensus amino acid sequence (SEQ ID NO:9) derived from a hidden Markov model is depicted in Figure 3F.

In a preferred embodiment 16836 polypeptide or protein of the disclosure has a "RA domain" or a region which includes at least about 50-140 more preferably about 80-120 or 90-110 amino acid residues and has at least about 50%, 60%, 70% 80% 90% 95%, 99%, or 100% homology with a "RA domain," e.g., a RA domain of human 16836 (e.g., residues 1640-1745 of SEQ ID NO:2).

To identify the presence of a "PI-PLC-X" domain, a "PI-PLC-Y" domain, a "C2" domain, or a "RA" domain in a 16836 protein sequence, and make the determination that a polypeptide or protein of interest has a particular profile, the amino acid sequence of the protein can be searched against the PFAM database of HMMs (e.g., the Pfam database, release 2.1) using the default parameters (http://www.sanger.ac.uk/Software/PFAM/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the PFAM database can be found in Sonhammer et al. (1997) Proteins 28(3):405-420 and a detailed description of HMMs can be found, for example, in Gribskov et al.(1990) Meth. Enzymol. 183:146-159; Gribskov et al.(1987) Proc. Natl. Acad. Sci. USA 84:4355-4358; Krogh et al.(1994) J. Mol. Biol. 235:1501-1531; and Stultz et al.(1993) Protein Sci. 2:305-314, the contents of which are incorporated herein by reference.

To identify the presence of a "RasGEF" domain in a 16836 protein sequence, and make the determination that a polypeptide or protein of interest has a particular profile, the amino acid sequence of the protein can be searched against a SMART database (Simple Modular Architecture Research Tool, http://smart.embl-heidelberg.de/) of HMMs as described in Schultz et al. (1998), Proc. Natl. Acad. Sci. USA 95:5857 and Schultz et al. (200) Nucl. Acids Res 28:231. The database contains domains identified by profiling with the hidden Markov models of the HMMer2 search program (R. Durbin et al. (1998) Biological sequence analysis: probabilistic models of proteins and nucleic acids. Cambridge University Press.; http://hmmer.wustl.edu/). The database also is extensively annotated and monitored by experts to enhance accuracy. A search was performed against the HMM database resulting in the identification of a "RasGEF" domain in the amino acid sequence of human 16836 at about residues 35 to 338 of SEQ ID NO:2 (see Figure 3A).

A 16836 family member of the disclosure can include a RasGEF domain, a PI-PLC-X domain, at least one and preferably two PI-PLC-Y domains, a C2 domain, and/or or a RA domain.

Furthermore, a 16836 family member of the disclosure can include at least one, two, three, four, five, six, seven, eight, nine, 10, 11, 12, 13, 14, 15, and preferably 16 N-glycosylation sites (PS00001); at least one, two, three, four, five, and preferably six cAMP- and cGMP-dependent protein kinase phosphorylation sites (PS00004); at least one, two, three, four, five, six, seven, eight, nine, 10, 11, 12, 13, 14, 15, 16, 17, 18, 19, 20, 21, 22, 23, 24, 25, 26, 27, 28, 29, and preferably 30 protein kinase C phosphorylation sites (PS00005); at least one, two, three, four, five, six, seven, eight, nine, 10, 11, 12, 13, 14, 15, 16, 17, 18, 19, 20, 21, 22, 23, 24, 25, 26, 27, 28, 29, 30, 31, 32, 33, 34, 35, 36, and preferably 37 casein kinase II phosphorylation sites (PS00006); at least one, two, three, four, five, six, seven, eight, nine, 10, 11, 12, 13, 14, 15, and preferably 16 N-myristoylation sites (PS00008); at least one amidation site (PS00009); and at least one coiled coil domain.

Activating mutations of the kras oncogene are common in both lung and colon tumors. Mutations in kras result in increased cellular proliferation, presumably mediated through effectors of ras function. 16836 appears to be a novel ras effector. An association of 16836 with the ras pathway is further supported by its increased expression in tumors (e.g., lung, breast, and colon tumors) and particularly in tumors with activating kras mutations. Expression of 16836 may be a required component of the kras signaling pathway in tumor cells, and increases in the levels of 16836 may contribute to the process of tumorigenesis.

As the 16836 polypeptides of the invention may modulate 16836-mediated activities, they may be useful as of for developing novel diagnostic and therapeutic agents for 16836-mediated or related disorders, as described below.

As used herein, a "16836 activity", "biological activity of 16836" or "functional activity of 16836," refers to an activity exerted by a 16836 protein, polypeptide or nucleic acid molecule. For example, a 16836 activity can be an activity exerted by 16836 in a physiological milieu on, e.g., a 16836-responsive cell or on a 16836 substrate, e.g., a protein substrate. A 16836 activity can be determined *in vivo* or *in vitro.* In one embodiment, a 16836 activity is a direct activity, such as an association with a 16836 target molecule. A "target molecule" or "binding partner" is a molecule with which a 16836 protein binds or interacts in nature, e.g., a phosphatidylinositol or a ras protein.

A 16836 activity can also be an indirect activity, e.g., a cellular signaling activity mediated by interaction of the 16836 protein with a 16836 ligand. Based on the above-described sequence similarities as well as 16836 expression patterns, the 16836 molecules of the present invention are predicted to have similar biological activities as phospholipase C family members and ras association proteins. Members of the phospholipase C family play important roles in signal transduction. An activated PLC is capable of catalyzing the hydrolysis of PIP₂, a minor component of the plasma membrane, to produce DAG and IP₃. IP₃ causes the release of calcium from intracellular stores and increases the influx of calcium from the extracellular fluid. The calcium ions directly regulate target enzymes and indirectly affect other enzymes by functioning as a second messenger and interacting with calcium-binding proteins, such as troponin C and calmodulin. For example, calcium ions regulate muscle contraction, glycogen breakdown and exocytosis. DAG, a product of hydrolysis by PI-PLCs, acts as a second messenger by activating protein kinase C. Activated protein kinase C phosphorylates a great number of intracellular proteins at the serine and threonine residues and modulates different signaling pathways. For example, the phosphorylation of glycogen synthase by protein kinase C stops the synthesis of glycogen. Moreover, protein kinase C controls cell division and proliferation. Both pathways are part of transmembrane signal transduction mechanisms, which regulate cellular processes, which include secretion, neural activity, metabolism, differentiation and proliferation. The presence of an RA domain in 16836 suggests that it is a ras effector, participating in propagating or amplifying cellular proliferation signals transduced by ras.

The16836 proteins of the present invention can have one or more of the following activities: (1) phospholipid metabolizing activity, e.g., the ability to catalyze hydrolysis of PIP₂ to produce DAG and IP₃; (2) the ability to associate with ras, e.g., activated ras; (3) the ability to propagate ras-mediated signal transduction; (4) the ability to mediate guanine nucleotide exchange activity; (5) the ability to transduce membrane signals; (6) the ability to modulate proliferation; (7) the ability to modulate differentiation; (8) the ability to modulate secretion; (9) the ability to modulate cell migration; (10) the ability to modulate metabolism; (11) the ability to modulate sensory perception; or (12) the ability to modulate fertilization.

The 16836 molecules can act as novel diagnostic targets and therapeutic agents for controlling one or more of cellular proliferative and/or differentiative disorders or disorders associated with bone metabolism.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the terms "cancer", "hyperproliferative" and "neoplastic" refer to cells having the capacity for autonomous growth. Examples of such cells include cells having an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genitourinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

Additional examples of proliferative disorders include hematopoietic neoplastic disorders. As used herein, the term "hematopoietic neoplastic disorders" includes diseases involving hyperplastic/neoplastic cells of hematopoietic origin. A hematopoietic neoplastic disorder can arise from myeloid, lymphoid or erythroid lineages, or precursor cells thereof. Preferably, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) Crit Rev. in Oncol./Hemotol. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Expression of 16836 mRNA is detected in bone cells (e.g., osteoclasts), testis, brain, skeletal muscle, breast, heart, and fetal liver (Figure 4). Accordingly, the molecules of the invention may mediate disorders involving aberrant activities of those cells, e.g., bone disorders, reproductive disorders, cardiovascular disorders, skeletal muscle disorders, or immune disorders as described in more detail below.

The presence of 16836 RNA or protein can be used to identify a cell or tissue, or other biological sample, as being derived from osteoclasts, testis, brain, skeletal muscle, breast, heart, or fetal liver, or being of human origin. Expression can be determined by evaluating RNA, e.g., by hybridization of a 16836 specific probe, or with a 16836 specific antibody.

The 16836 protein, fragments thereof, and derivatives and other variants of the sequence in SEQ ID NO:2 thereof are collectively referred to as "polypeptides or proteins of the invention" or "16836 polypeptides or proteins". Nucleic acid molecules encoding such polypeptides or proteins are collectively referred to as "nucleic acids of the invention" or "16836 nucleic acids." 16836 molecules refer to 16836 nucleic acids, polypeptides, and antibodies.

As used herein, the term "nucleic acid molecule" includes DNA molecules (e.g., a cDNA or genomic DNA), RNA molecules (e.g., an mRNA) and analogs of the DNA or RNA. A DNA or RNA analog can be synthesized from nucleotide analogs. The nucleic acid molecule can be single-stranded or double-stranded, but preferably is double-stranded DNA.

The term "isolated nucleic acid molecule" or "purified nucleic acid molecule" includes nucleic acid molecules that are separated from other nucleic acid molecules present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

Preferably, an isolated nucleic acid molecule of the invention that hybridizes under a stringency condition described herein to the sequence of SEQ ID NO:1 or SEQ ID NO:3, corresponds to a naturally-occurring nucleic acid molecule.

As used herein, a "naturally-occurring" nucleic acid molecule refers to an RNA or DNA molecule having a nucleotide sequence that occurs in nature. For example a naturally occurring nucleic acid molecule can encode a natural protein.

As used herein, the terms "gene" and "recombinant gene" refer to nucleic acid molecules which include at least an open reading frame encoding a 16836 protein. The gene can optionally further include non-coding sequences, e.g., regulatory sequences and introns. Preferably, a gene encodes a mammalian 16836 protein or derivative thereof

An "isolated" or "purified" polypeptide or protein is substantially free of cellular material or other contaminating proteins from the cell or tissue source from which the protein is derived, or substantially free from chemical precursors or other chemicals when chemically synthesized. "Substantially free" means that a preparation of 16836 protein is at least 10% pure. In a preferred embodiment, the preparation of 16836 protein has less than about 30%, 20%, 10% and more preferably 5% (by dry weight), of non-16836 protein (also referred to herein as a "contaminating protein"), or of chemical precursors or non-16836 chemicals. When the 16836 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 16836 without abolishing or substantially altering a 16836 activity. Preferably the alteration does not substantially alter the 16836 activity, e.g., the activity is at least 20%, 40%, 60%, 70% or 80% of wild-type. An "essential" amino acid residue is a residue that, when altered from the wild-type sequence of 16836, results in abolishing a 16836 activity such that less than 20% of the wild-type activity is present. For example, conserved amino acid residues in 16836 are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 16836 protein is preferably replaced with another amino acid residue from the same side chain family. Alternatively, in another embodiment, mutations can be introduced randomly along all or part of a 16836 coding sequence, such as by saturation mutagenesis, and the resultant mutants can be screened for 16836 biological activity to identify mutants that retain activity. Following mutagenesis of SEQ ID NO:1 or SEQ ID NO:3, the encoded protein can be expressed recombinantly and the activity of the protein can be determined.

As used herein, a "biologically active portion" of a 16836 protein includes a fragment of a 16836 protein which participates in an interaction, e.g., an intramolecular or an inter-molecular interaction. An inter-molecular interaction can be a specific binding interaction or an enzymatic interaction (e.g., the interaction can be transient and a covalent bond is formed or broken). An inter-molecular interaction can be between a 16836 molecule and a non-16836 molecule or between a first 16836 molecule and a second 16836 molecule (e.g., a dimerization interaction). Biologically active portions of a 16836 protein include peptides comprising amino acid sequences sufficiently homologous to or derived from the amino acid sequence of the 16836 protein, e.g., the amino acid sequence shown in SEQ ID NO:2, which include less amino acids than the full length 16836 proteins, and exhibit at least one activity of a 16836 protein. Typically, biologically active portions comprise a domain or motif with at least one activity of the 16836 protein, e.g., the ability to catalyze the hydrolysis of phosphatidylinositol or associate with Ras. A biologically active portion of a 16836 protein can be a polypeptide which is, for example, 10, 25, 50, 100, 200 or more amino acids in length. Biologically active portions of a 16836 protein can be used as targets for developing agents which modulate a 16836 mediated activity, e.g., the ability to catalyze the hydrolysis of phosphatidylinositol or associate with Ras.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol Biol. 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm ofE. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to 16836 nucleic acid molecules of the invention. BLAST protein searches can be performed with the XBLAST program, score = 50, wordlength = 3 to obtain amino acid sequences homologous to 16836 protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov.

Particular 16836 polypeptides of the present invention have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO:1 or 3 are termed substantially identical.

"Misexpression or aberrant expression", as used herein, refers to a non-wildtype pattern of gene expression at the RNA or protein level. It includes: expression at non-wild type levels, i.e., over- or under-expression; a pattern of expression that differs from wild type in terms of the time or stage at which the gene is expressed, e.g., increased or decreased expression (as compared with wild type) at a predetermined developmental period or stage; a pattern of expression that differs from wild type in terms of altered, e.g., increased or decreased, expression (as compared with wild type) in a predetermined cell type or tissue type; a pattern of expression that differs from wild type in terms of the splicing size, translated amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, refers to human and non-human animals. The term "non-human animals" of the invention includes all vertebrates, e.g., mammals, such as non-human primates (particularly higher primates), sheep, dog, rodent (e.g., mouse or rat), guinea pig, goat, pig, cat, rabbits, cow, and non-mammals, such as chickens, amphibians, reptiles, etc. In a preferred embodiment, the subject is a human. In another embodiment, the subject is an experimental animal or animal suitable as a disease model.

A "purified preparation of cells", as used herein, refers to an in vitro preparation of cells. In the case cells from multicellular organisms (e.g., plants and animals), a purified preparation of cells is a subset of cells obtained from the organism, not the entire intact organism. In the case of unicellular microorganisms (e.g., cultured cells and microbial cells), it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 16836 polypeptide described herein, e.g., a full-length 16836 protein or a fragment thereof, e.g., a biologically active portion of 16836 protein. Also included is a nucleic acid fragment suitable for use as a hybridization probe, which can be used, e.g., to identify a nucleic acid molecule encoding a polypeptide of the invention, 16836 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:1, or a portion of any of these nucleotide sequences. In one embodiment, the nucleic acid molecule includes sequences encoding the human 16836 protein (i.e., "the coding region" of SEQ ID NO:1, as shown in SEQ ID NO:3), as well as 5' untranslated sequences. Alternatively, the nucleic acid molecule can include only the coding region of SEQ ID NO:1 (e.g., SEQ ID NO:3) and, e.g., no flanking sequences which normally accompany the subject sequence. The disclosure also encompasses where, the nucleic acid molecule encodes a sequence corresponding to a fragment of the protein from about amino acid 35-338, 900-1048, 1171-1184, 1261-1353, 1378-1460, or 1640-1745 of SEQ ID NO:2.

In another embodiment, an isolated nucleic acid molecule of the invention includes a nucleic acid molecule which is a complement of the nucleotide sequence shown in SEQ ID NO: I₋or SEQ ID NO:3, or a portion of any of these nucleotide sequences. In other embodiments, the nucleic acid molecule of the invention is sufficiently complementary to the nucleotide sequence shown in SEQ ID NO:1 or SEQ ID NO:3, such that it can hybridize (e.g., under a stringency condition described herein) to the nucleotide sequence shown in SEQ ID NO: 1 or 3, thereby forming a stable duplex.

In one embodiment, an isolated nucleic acid molecule of the present invention includes a nucleotide sequence which is at least about: 80%, 85%, 90%, 91%, 92%, 93%, 94%, 95%, 96%, 97%, 98%, 99%, or more homologous to the entire length of the nucleotide sequence shown in SEQ ID NO:1 or SEQ ID NO:3, or a portion, preferably of the same length, of any of these nucleotide sequences.

### 16836 Nucleic Acid Fragments

A nucleic acid molecule of the invention can include only a portion of the nucleic acid sequence of SEQ ID NO:1 or 3. For example, such a nucleic acid molecule can include a fragment which can be used as a probe or primer or a fragment encoding a portion of a 16836 protein, e.g., an immunogenic or biologically active portion of a 16836 protein. A fragment can comprise those nucleotides of SEQ ID NO:1 which encode a phospholipase C, RA, or RasGEF domain of human 16836. The nucleotide sequence determined from the cloning of the 16836 gene allows for the generation of probes and primers designed for use in identifying and/or cloning other 16836 family members, or fragments thereof, as well as 16836 homologues, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 100 amino acids in length, e.g., at least 1200, 1400, 1600, or 1800 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 16836 nucleic acid fragment can include a sequence corresponding to a phospholipase C, RA, or RasGEF domain.

16836 probes and primers are provided. Typically a probe/primer is an isolated or purified oligonucleotide. The oligonucleotide typically includes a region of nucleotide sequence that hybridizes under a stringency condition described herein to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO:1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: a Ras guanine nucleotide exchange factor (RasGEF) domain (about amino acid residues 35-338 of SEQ ID NO:2); a phosphatidylinositol-specific phospholipase C "X" (PI-PLC-X) domain (about amino acid residues 900-1048 of SEQ ID NO:2); a phosphatidylinositol-specific phospholipase C "Y" (PI-PLC-Y) domain (about amino acid residues 1171-1184 or 1261-1353 of SEQ ID NO:2); a C2 domain (about amino acid residues 1378-1460 of SEQ ID NO:2); or a Ras association (RaIGDS/AF-6) (RA) domain (about amino acid residues 1640-1745 of SEQ ID NO:2).

In another embodiment a set of primers is provided, e.g., primers suitable for use in a PCR, which can be used to amplify a selected region of a 16836 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variant. For example, primers suitable for amplifying all or a portion of any of the following regions are provided: a Ras guanine nucleotide exchange factor (RasGEF) domain (about amino acid residues 35-338 of SEQ ID NO:2); a phosphatidylinositol-specific phospholipase C "X" (PI-PLC-X) domain (about amino acid residues 900-1048 of SEQ ID NO:2); a phosphatidylinositol-specific phospholipase C "Y" (PI-PLC-Y) domain (about amino acid residues 1171-1184 or 1261-1353 of SEQ ID NO:2); a C2 domain (about amino acid residues 1378-1460 of SEQ ID NO:2); or a Ras association (RaIGDS/AF-6) (RA) domain (about amino acid residues 1640-1745 of SEQ ID NO:2).

A nucleic acid fragment can encode an epitope bearing region of a polypeptide described herein.

A nucleic acid fragment encoding a "biologically active portion of a 16836 polypeptide" can be prepared by isolating a portion of the nucleotide sequence of SEQ ID NO:1 or 3, which encodes a polypeptide having a 16836 biological activity (e.g., the biological activities of the 16836 proteins are described herein), expressing the encoded portion of the 16836 protein (e.g., by recombinant expression *in vitro*) and assessing the activity of the encoded portion of the 16836 protein. For example, a nucleic acid fragment encoding a biologically active portion of 16836 includes a phospholipase C, RA, or RasGEF domain, e.g., amino acid residues about 35-338, 900-1048, 1171-1184, 1261-1353, 1378-1460, or 1640-1745 of SEQ ID NO:2. A nucleic acid fragment encoding a biologically active portion of a 16836 polypeptide, may comprise a nucleotide sequence which is greater than 300 or more nucleotides in length.

In preferred embodiments, the nucleic acid fragment includes a nucleotide sequence that is other than the sequence of AF117948, AL050031, AW243053, AW272589, AW272590, A02325, V89699, AC03885, or WO/58473.

In preferred embodiments, the fragment comprises the coding region of 16836, e.g., the nucleotide sequence of SEQ ID NO:3.

In preferred embodiments, a nucleic acid includes a nucleotide sequence which is about 4000, 4500, 5000, 6000, 7000, 8000, 9000, 10000, or more nucleotides in length and hybridizes under a stringency condition described herein to a nucleic acid molecule of SEQ ID NO:1, or SEQ ID NO:3.

### 16836 Nucleic Acid Variants

The disclosure further encompasses nucleic acid molecules that differ from the nucleotide sequence shown in SEQ ID NO:1 or SEQ ID NO:3. Such differences can be due to degeneracy of the genetic code (and result in a nucleic acid which encodes the same 16836 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. "Looped" out sequences from deletions or insertions, or mismatches, are considered differences.

Nucleic acids of the disclosure can be chosen for having codons, which are preferred, or non-preferred, for a particular expression system. E.g., the nucleic acid can be one in which at least one codon, at preferably at least 10%, or 20% of the codons has been altered such that the sequence is optimized for expression in E. *coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under a stringency condition described herein, to the nucleotide sequence shown in SEQ ID NO 2 or a fragment of the sequence. Nucleic acid molecules corresponding to orthologs, homologs, and allelic variants of the 16836 cDNAs of the invention can further be isolated by mapping to the same chromosome or locus as the 16836 gene.

Preferred variants include those that are correlated with the ability to catalyze the hydrolysis of phosphatidylinositol or associate with Ras.

Allelic variants of 16836, e.g., human 16836, include both functional and non-functional proteins. Functional allelic variants are naturally occurring amino acid sequence variants of the 16836 protein within a population that maintain the ability to catalyze the hydrolysis of phosphatidylinositol or associate with Ras. Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 16836, e.g., human 16836, protein within a population that do not have the ability to catalyze the hydrolysis of phosphatidylinositol or associate with Ras. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

### Antisense Nucleic Acid Molecules, Ribozymes and Modified 16836 Nucleic Acid Molecules

In another aspect, the invention features, an isolated nucleic acid molecule which is antisense to 16836. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 16836 coding strand, or to only a portion thereof (e.g., the coding region of human 16836 corresponding to SEQ ID NO:3). In another embodiment, the antisense nucleic acid molecule is antisense to a "noncoding region" of the coding strand of a nucleotide sequence encoding 16836 (e.g., the 5' and 3' untranslated regions).

An antisense nucleic acid can be designed such that it is complementary to the entire coding region of 16836 mRNA, but more preferably is an oligonucleotide which is antisense to only a portion of the coding or noncoding region of 16836 mRNA. For example, the antisense oligonucleotide can be complementary to the region surrounding the translation start site of 16836 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 16836 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

In still another embodiment, an antisense nucleic acid of the invention is a ribozyme. A ribozyme having specificity for a 16836-encoding nucleic acid can include one or more sequences complementary to the nucleotide sequence of a 16836 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) Nature 334:585-591). For example, a derivative of a *Tetrahymena* L-19 IVS RNA can be constructed in which the nucleotide sequence of the active site is complementary to the nucleotide sequence to be cleaved in a 16836-encoding mRNA. See, e.g., Cech et al. U.S. Patent No. 4,987,071; and Cech et al. U.S. Patent No. 5,116,742. Alternatively, 16836 mRNA can be used to select a catalytic RNA having a specific ribonuclease activity from a pool of RNA molecules. See, e.g., Bartel, D. and Szostak, J. W. (1993) Science 261:1411-1418.

16836 gene expression can be inhibited by targeting nucleotide sequences complementary to the regulatory region of the 16836 (e.g., the 16836 promoter and/or enhancers) to form triple helical structures that prevent transcription of the 16836 gene in target cells. See generally, Helene, C. (1991) Anticancer Drug Des. 6:569-84; Helene, C. i (1992) Ann. N.Y. Acad Sci. 660:27-36; and Maher, L.J. (1992) Bioassays 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

### Isolated 16836 Polypeptides

In another aspect, the invention features, an isolated 16836 protein, or fragment, e.g., a biologically active portion, for use as immunogens or antigens to raise or test (or more generally to bind) anti-16836 antibodies. 16836 protein can be isolated from cells or tissue sources using standard protein purification techniques. 16836 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when expressed the polypeptide is expressed in a native cell, or in systems which result in the alteration or omission of post-translational modifications, e.g., glycosylation or cleavage, present when expressed in a native cell.

In a preferred embodiment, a 16836 polypeptide has one or more of the following characteristics:
(i) it has the ability to catalyze the hydrolysis of phosphatidylinositol;
(ii) it has the ability to associate with ras, preferably activated (GTP-bound) ras;
(iii) it has the ability to mediate guanine nucleotide exchange activity;
(iv) it has a molecular weight, e.g., a deduced molecular weight, preferably ignoring any contribution of post translational modifications, amino acid composition or other physical characteristic of a 16836 polypeptide, e.g., a polypeptide of SEQ ID NO:2;
(v) it has an overall sequence similarity of at least 80, 90, or 95%, with a polypeptide of SEQ ID NO:2;
(vi) it can be found in bone cells, fetal liver cells, brain cells, skeletal muscle, testis, breast, or heart;
(vii) it has a RasGEF domain with an overall sequence similarity of about 70%, 80%, 90% or 95% with amino acid residues 35-338 of SEQ ID NO:2;
(viii) it has a PI-PLC-X domain with an overall sequence similarity of about 70%, 80%, 90% or 95% with amino acid residues 900-1048 of SEQ ID NO:2;
(ix) it has a PI-PLC-Y domain with an overall sequence similarity of about 70%, 80%, 90% or 95% with amino acid residues 1171-1184 or 1261-1353 of SEQ ID NO:2;
(x) it has a C2 domain with an overall sequence similarity of about 70%, 80%, 90% or 95% with amino acid residues 1378-1460 of SEQ ID NO:2;
(xi) it has a RA domain with an overall sequence similarity of about 70%, 80%, 90% or 95% with amino acid residues 1640-1745 of SEQ ID NO:2;
(xii) it can colocalize with the ras protein; or
(xiii) it has at least 70%, preferably 80%, and most preferably 90% of the cysteines found amino acid sequence of the native protein.

In a preferred embodiment the 16836 protein, or fragment thereof, differs from the corresponding sequence in SEQ ID:2. In one embodiment it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a non essential residue or a conservative substitution. In a preferred embodiment the differences are not in the phospholipase C, RA, or RasGEF domains. In another preferred embodiment one or more differences are in the phospholipase C, RA, or RasGEF domains.

Other embodiments include a protein that contain one or more changes in amino acid sequence, e.g., a change in an amino acid residue which is not essential for activity. Such 16836 proteins differ in amino acid sequence from SEQ ID NO:2, yet retain biological activity.

In one embodiment, the protein includes an amino acid sequence at least about 80%, 85%, 90%, 95%, 98% or more homologous to SEQ ID NO:2.

A 16836 protein or fragment is provided which varies from the sequence of SEQ ID NO:2 in regions defined by amino acids about 1-57, 233-899, 1049-1170, 1185-1260, 1354-1377, 1461-1639, or 1746-1809 of SEQ ID NO:2 by at least one but by less than 15, 10 or 5 amino acid residues in the protein or fragment but which does not differ from SEQ ID NO:2 in regions defined by amino acids about 35-338, 900-1048, 1171-1184, 1261-1353, 1378-1460, or 1640-1745 of SEQ ID NO:2. (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 16836 protein includes a phospholipase C, RA, or RasGEF domain. Moreover, other biologically active portions, in which other regions of the protein are deleted, can be prepared by recombinant techniques and evaluated for one or more of the functional activities of a native 16836 protein.

In a preferred embodiment, the 16836 protein has an amino acid sequence shown in SEQ ID NO:2. In other embodiments, the 16836 protein is substantially identical to SEQ ID NO:2. In yet another embodiment, the 16836 protein is substantially identical to SEQ ID NO:2 and retains the functional activity of the protein of SEQ ID NO:2, as described in detail in the subsections above.

### 16836 Chimeric or Fusion Proteins

In another aspect, the invention provides 16836 chimeric or fusion proteins. As used herein, a 16836 "chimeric protein" or "fusion protein" includes a 16836 polypeptide linked to a non-16836 polypeptide. A "non-16836 polypeptide" refers to a polypeptide having an amino acid sequence corresponding to a protein which is not substantially homologous to the 16836 protein, e.g., a protein which is different from the 16836 protein and which is derived from the same or a different organism. The 16836 polypeptide of the fusion protein can correspond to all or a portion e.g., a fragment described herein of a 16836 amino acid sequence. In a preferred embodiment, a 16836 fusion protein includes at least one (or two) biologically active portion of a 16836 protein. The non-16836 polypeptide can be fused to the N-terminus or C-terminus of the 16836 polypeptide.

The fusion protein can include a moiety which has a high affinity for a ligand. For example, the fusion protein can be a GST-16836 fusion protein in which the 16836 sequences are fused to the C-terminus of the GST sequences. Such fusion proteins can facilitate the purification of recombinant 16836. Alternatively, the fusion protein can be a 16836 protein containing a heterologous signal sequence at its N-terminus. In certain host cells (e.g., mammalian host cells), expression and/or secretion of 16836 can be increased through use of a heterologous signal sequence.

Fusion proteins can include all or a part of a serum protein, e.g., an IgG constant region, or human serum albumin.

The 16836 fusion proteins of the invention can be incorporated into pharmaceutical compositions and administered to a subject *in vivo.* The 16836 fusion proteins can be used to affect the bioavailability of a 16836 substrate. 16836 fusion proteins may be useful therapeutically for the treatment of disorders caused by, for example, (i) aberrant modification or mutation of a gene encoding a 16836 protein; (ii) mis-regulation of the 16836 gene; and (iii) aberrant post-translational modification of a 16836 protein.

Moreover, the 16836-fusion proteins of the invention can be used as immunogens to produce anti-16836 antibodies in a subject, to purify 16836 ligands and in screening assays to identify molecules that inhibit the interaction of 16836 with a 16836 substrate.

Expression vectors are commercially available that already encode a fusion moiety (e.g., a GST polypeptide). A 16836-encoding nucleic acid can be cloned into such an expression vector such that the fusion moiety is linked in-frame to the 16836 protein.

### Anti-16836 Antibodies

In another aspect, the invention provides an anti-16836 antibody, or a fragment thereof (e.g., an antigen-binding fragment thereof). The term "antibody" as used herein refers to an immunoglobulin molecule or immunologically active portion thereof, i.e., an antigen-binding portion. As used herein, the term "antibody" refers to a protein comprising at least one, and preferably two, heavy (H) chain variable regions (abbreviated herein as VH), and at least one and preferably two light (L) chain variable regions (abbreviated herein as VL). The VH and VL regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat, E.A, et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917, which are incorporated herein by reference). Each VH and VL is composed of three CDR's and four FRs, arranged from amino-terminus to carboxy-terminus in the following order: FR1, CDR1, FR2, CDR2, FR3, CDR3, FR4.

The anti-16836 antibody can further include a heavy and light chain constant region, to thereby form a heavy and light immunoglobulin chain, respectively. In one embodiment, the antibody is a tetramer of two heavy immunoglobulin chains and two light immunoglobulin chains, wherein the heavy and light immunoglobulin chains are inter-connected by, e.g., disulfide bonds. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

As used herein, the term "immunoglobulin" refers to a protein consisting of one or more polypeptides substantially encoded by immunoglobulin genes. The recognized human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin "light chains" (about 25 KDa or 214 amino acids) are encoded by a variable region gene at the NH2-terminus (about 110 amino acids) and a kappa or lambda constant region gene at the COOH-terminus. Full-length immunoglobulin "heavy chains" (about 50 KDa or 446 amino acids), are similarly encoded by a variable region gene (about 116 amino acids) and one of the other aforementioned constant region genes, e.g., gamma (encoding about 330 amino acids).

The term "antigen-binding fragment" of an antibody (or simply "antibody portion," or "fragment"), as used herein, refers to one or more fragments of a full-length antibody that retain the ability to specifically bind to the antigen, e.g., 16836 polypeptide or fragment thereof. Examples of antigen-binding fragments of the anti-16836 antibody include, but are not limited to: (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341 :544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad Sci. USA 85:5879-5883). Such single chain antibodies are also encompassed within the term "antigen-binding fragment" of an antibody. These antibody fragments are obtained using conventional techniques known to those with skill in the art, and the fragments are screened for utility in the same manner as are intact antibodies.

The anti-16836 antibody can be a polyclonal or a monoclonal antibody. In other embodiments, the antibody can be recombinantly produced, e.g., produced by phage display or by combinatorial methods.

Phage display and combinatorial methods for generating anti-16836 antibodies are known in the art (as described in, e.g., Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982, the contents of all of which are incorporated by reference herein).

In one embodiment, the anti-16836 antibody is a fully human antibody (e.g., an antibody made in a mouse which has been genetically engineered to produce an antibody from a human immunoglobulin sequence), or a non-human antibody, e.g., a rodent (mouse or rat), goat, primate (e.g., monkey), camel antibody. Preferably, the non-human antibody is a rodent (mouse or rat antibody). Method of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An anti-16836 antibody can be one in which the variable region, or a portion thereof, e.g., the CDR's, are generated in a non-human organism, e.g., a rat or mouse. Chimeric, CDR-grafted, and humanized antibodies are within the invention. Antibodies generated in a non-human organism, e.g., a rat or mouse, and then modified, e.g., in the variable framework or constant region, to decrease antigenicity in a human are within the invention.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).
A humanized or CDR-grafted antibody will have at least one or two but generally all three recipient CDR's (of heavy and or light immuoglobulin chains) replaced with a donor CDR An antibody may be replaced with at least a portion of a non-human CDR or only some of the CDR's may be replaced with non-human CDR's. It is only necessary to replace the number of CDR's required for binding of the humanized antibody to a 16836 or a fragment thereof Preferably, the donor will be a rodent antibody, e.g., a rat or mouse antibody, and the recipient will be a human framework or a human consensus framework. Typically, the immunoglobulin providing the CDR's is called the "donor" and the immunoglobulin providing the framework is called the "acceptor." In one embodiment, the donor immunoglobulin is a non-human (e.g., rodent). The acceptor framework is a naturally-occurring (e.g., a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See e.g., Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. US 5,585,089, US 5,693,761 and US 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a 16836 polypeptide or fragment thereof The recombinant DNA encoding the humanized antibody, or fragment thereof, can then be cloned into an appropriate expression vector.

Humanized or CDR-grafted antibodies can be produced by CDR-grafting or CDR substitution, wherein one, two, or all CDR's of an immunoglobulin chain can be replaced. See e.g., U.S. Patent 5,225,539; Jones et al. 1986 Nature 321:552-525; Verhoeyan et al. 1988 Science 239:1534; Beidler et al. 1988 J. Immunol. 141:4053-4060; Winter US 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US 5,225,539), the contents of which is expressly incorporated by reference.

Also within the scope of the invention are humanized antibodies in which specific amino acids have been substituted, deleted or added. Preferred humanized antibodies have amino acid substitutions in the framework region, such as to improve binding to the antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR (see e.g., US 5,585,089). Criteria for selecting amino acids from the donor are described in US 5,585,089, e.g., columns 12-16 of US 5,585,089, the e.g., columns 12-16 of US 5,585,089, the contents of which are hereby incorporated by reference. Other techniques for humanizing antibodies are described in Padlan et al. EP 519596 A1, published on December 23, 1992.

In preferred embodiments an antibody can be made by immunizing with purified 16836 antigen, or a fragment thereof, e.g., a fragment described herein, tissue, e.g., crude tissue preparations, whole cells, preferably living cells, lysed cells, or cell fractions.

A full-length 16836 protein or, antigenic peptide fragment of 16836 can be used as an immunogen or can be used to identify anti-16836 antibodies made with other immunogens, e.g., cells, membrane preparations, and the like. The antigenic peptide of 16836 should include at least 8 amino acid residues of the amino acid sequence shown in SEQ ID NO:2 and encompasses an epitope of 16836. Preferably, the antigenic peptide includes at least 10 amino acid residues, more preferably at least 15 amino acid residues, even more preferably at least 20 amino acid residues, and most preferably at least 30 amino acid residues.

Fragments of 16836 which include residues about 330-350, about 610-630, or about 1120-1140 can be used to make, e.g., used as immunogens or used to characterize the specificity of an antibody, antibodies against hydrophilic regions of the 16836 protein. Similarly, fragments of 16836 which include residues about 200-230, about 430-450, or about 830-840 can be used to make an antibody against a hydrophobic region of the 16836 protein. A fragment of 16836 which includes residues about 35-338 can be used to make an antibody against the RasGEF region of the 16836 protein. A fragment of 16836 which includes residues about 35-338 can be used to make an antibody against the RasGEF region of the 16836 protein. A fragment of 16836 which includes residues about 900-1048 can be used to make an antibody against the PI-PLC-X region of the 16836 protein. A fragment of 16836 which includes residues about 1171-1184 or 1261-1353 can be used to make an antibody against the PI-PLC-Y region of the 16836 protein. A fragment of 16836 which includes residues about 1378-1460 can be used to make an antibody against the C2 region of the 16836 protein. A fragment of 16836 which includes residues about 1640-1745 can be used to make an antibody against the RA region of the 16836 protein.

Antibodies reactive with, or specific for, any of these regions, or other regions or domains described herein are provided.

Antibodies which bind only native 16836 protein, only denatured or otherwise non-native 16836 protein, or which bind both, are with in the invention. Antibodies with linear or conformational epitopes are within the invention. Conformational epitopes can sometimes be identified by identifying antibodies which bind to native but not denatured 16836 protein.

Preferred epitopes encompassed by the antigenic peptide are regions of 16836 are located on the surface of the protein, e.g., hydrophilic regions, as well as regions with high antigenicity. For example, an Emini surface probability analysis of the human 16836 protein sequence can be used to indicate the regions that have a particularly high probability of being localized to the surface of the 16836 protein and are thus likely to constitute surface residues useful for targeting antibody production.

In preferred embodiments antibodies can bind one or more of purified antigen, tissue, e.g., tissue sections, whole cells, preferably living cells, lysed cells, or cell fractions.

The anti-16836 antibody can be a single chain antibody. A single-chain antibody (scFV) may be engineered (see, for example, Colcher, D. et al. (1999) Ann N YAcad Sci 880:263-80; and Reiter, Y. (1996) Clin Cancer Res 2:245-52). The single chain antibody can be dimerized or multimerized to generate multivalent antibodies having specificities for different epitopes of the same target 16836 protein.

In a preferred embodiment the antibody has: effector function; and can fix complement. In other embodiments the antibody does not; recruit effector cells; or fix complement.

In a preferred embodiment, the antibody has reduced or no ability to bind an Fc receptor. For example., it is a isotype or subtype, fragment or other mutant, which does not support binding to an Fc receptor, e.g., it has a mutagenized or deleted Fc receptor binding region.

In a preferred embodiment, an anti-16836 antibody alters (e.g., increases or decreases) the ability of a 16836 polypeptide to catalyze the hydrolysis of phosphatidylinositol or to associate with Ras.

The antibody can be coupled to a toxin, e.g., a polypeptide toxin, e,g, ricin or diphtheria toxin or active fragment hereof, or a radioactive nucleus, or imaging agent, e.g. a radioactive, enzymatic, or other, e.g., imaging agent, e.g., a NMR contrast agent. Labels which produce detectable radioactive emissions or fluorescence are preferred.

An anti-16836 antibody (e.g., monoclonal antibody) can be used to isolate 16836 by standard techniques, such as affinity chromatography or immunoprecipitation. Moreover, an anti-16836 antibody can be used to detect 16836 protein (e.g., in a cellular lysate or cell supernatant) in order to evaluate the abundance and pattern of expression of the protein. Anti-16836 antibodies can be used diagnostically to monitor protein levels in tissue as part of a clinical testing procedure, e.g., to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

The invention also includes a nucleic acid that encodes an anti-16836 antibody, e.g., an anti-16836 antibody described herein. Also included are vectors which include the nucleic acid and cells transformed with the nucleic acid, particularly cells which are useful for producing an antibody, e.g., mammalian cells, e.g. CHO or lymphatic cells.

The invention also includes cell lines, e.g., hybridomas, which make an anti-16836 antibody, e.g., and antibody described herein, and method of using said cells to make a 16836 antibody.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 16836 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 16836 proteins, mutant forms of 16836 proteins, fusion proteins, and the like).

The recombinant expression vectors of the invention can be designed for expression of 16836 proteins in prokaryotic or eukaryotic cells. For example, polypeptides of the invention can be expressed in *E. coli,* insect cells (e.g., using baculovirus expression vectors), yeast cells or mammalian cells. Suitable host cells are discussed further in Goeddel, (1990) Gene Expression Technology: Methods in Enzymology 185, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K. S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 16836 activity assays, (e.g., direct assays or competitive assays described in detail below), or to generate antibodies specific for 16836 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in E. *coli* is to express the protein in a host bacteria with an impaired capacity to proteolytically cleave the recombinant protein (Gottesman, S., (1990) Gene Expression Technology: Methods in Enzymology 185, Academic Press, San Diego, California 119-128). Another strategy is to alter the nucleic acid sequence of the nucleic acid to be inserted into an expression vector so that the individual codons for each amino acid are those preferentially utilized in E. *coli* (Wada et al., (1992) Nucleic Acids Res. 20:2111-2118). Such alteration of nucleic acid sequences of the invention can be carried out by standard DNA synthesis techniques.

The 16836 expression vector can be a yeast expression vector, a vector for expression in insect cells, e.g., a baculovirus expression vector or a vector suitable for expression in mammalian cells.

When used in mammalian cells, the expression vector's control functions can be provided by viral regulatory elements. For example, commonly used promoters are derived from polyoma, Adenovirus 2, cytomegalovirus and Simian Virus 40.

In another embodiment, the promoter is an inducible promoter, e.g., a promoter regulated by a steroid hormone, by a polypeptide hormone (e.g., by means of a signal transduction pathway), or by a heterologous polypeptide (e.g., the tetracycline-inducible systems, "Tet-On" and "Tet-Off"; see, e.g., Clontech Inc., CA, Gossen and Bujard (1992) Proc. Natl. Acad Sci. USA 89:5547, and Paillard (1989) Human Gene Therapy 9:983*).*

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) Science 249:374-379) and the α-fetoprotein promoter (Campes and Tilghman (1989) Genes Dev. 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus.

Another aspect the invention provides a host cell which includes a nucleic acid molecule described herein, e.g., a 16836 nucleic acid molecule within a recombinant expression vector or a 16836 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 16836 protein can be expressed in bacterial cells (such as *E*. *coli),* insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

A host cell of the invention can be used to produce (i.e., express) a 16836 protein. Accordingly, the invention further provides methods for producing a 16836 protein using the host cells of the invention. In one embodiment, the method includes culturing the host cell of the invention (into which a recombinant expression vector encoding a 16836 protein has been introduced) in a suitable medium such that a 16836 protein is produced. In another embodiment, the method further includes isolating a 16836 protein from the medium or the host cell.

In another aspect, the invention features, a cell or purified preparation of cells which include a 16836 transgene, or which otherwise misexpress 16836. The cell preparation can consist of human or non-human cells, e.g., rodent cells, e.g., mouse or rat cells, rabbit cells, or pig cells. In preferred embodiments, the cell or cells include a 16836 transgene, e.g., a heterologous form of a 16836, e.g., a gene derived from humans (in the case of a non-human cell). The 16836 transgene can be misexpressed, e.g., overexpressed or underexpressed. In other preferred embodiments, the cell or cells include a gene that mis-expresses an endogenous 16836, e.g., a gene the expression of which is disrupted, e.g., a knockout. Such cells can serve as a model for studying disorders that are related to mutated or mis-expressed 16836 alleles or for use in drug screening.

In another aspect, the invention features, a human cell, e.g., a hematopoietic stem cell, transformed with nucleic acid that encodes a subject 16836 polypeptide.

Also provided are cells, preferably human cells, e.g., human hematopoietic or fibroblast cells, in which an endogenous 16836 is under the control of a regulatory sequence that does not normally control the expression of the endogenous 16836 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 16836 gene. For example, an endogenous 16836 gene which is "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

In a preferred embodiment, recombinant cells described herein can be used for replacement therapy in a subject. For example, a nucleic acid encoding a 16836 polypeptide operably linked to an inducible promoter (e.g., a steroid hormone receptor-regulated promoter) is introduced into a human or nonhuman, e.g., mammalian, e.g., porcine recombinant cell. The cell is cultivated and encapsulated in a biocompatible material, such as poly-lysine alginate, and subsequently implanted into the subject. See, e.g., Lanza (1996) Nat. Biotechnol. 14:1107; Joki et al. (2001) Nat. Biotechnol. 19:35; and U.S. Patent No. 5,876,742. Production of 16836 polypeptide can be regulated in the subject by administering an agent (e.g., a steroid hormone) to the subject. In another preferred embodiment, the implanted recombinant cells express and secrete an antibody specific for a 16836 polypeptide. The antibody can be any antibody or any antibody derivative described herein.

### Uses

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express a 16836 protein (e.g., via a recombinant expression vector in a host cell in gene therapy applications), to detect a 16836 mRNA (e.g., in a biological sample) or a genetic alteration in a 16836 gene, and to modulate 16836 activity, as described further below. The 16836 proteins can be used to treat disorders characterized by insufficient or excessive production of a 16836 substrate or production of 16836 inhibitors. In addition, the 16836 proteins can be used to screen for naturally occurring 16836 substrates, to screen for drugs or compounds which modulate 16836 activity, as well as to treat disorders characterized by insufficient or excessive production of 16836 protein or production of 16836 protein forms which have decreased, aberrant or unwanted activity compared to 16836 wild type protein (e.g., cellular proliferative and/or differentiative disorders). Moreover, the anti-16836 antibodies of the invention can be used to detect and isolate 16836 proteins, regulate the bioavailability of 16836 proteins, and modulate 16836 activity.

A method of evaluating a compound for the ability to interact with, e.g., bind, a subject 16836 polypeptide is provided. The method includes: contacting the compound with the subject 16836 polypeptide; and evaluating ability of the compound to interact with, e.g., to bind or form a complex with the subj ect 16836 polypeptide. This method can be performed in vitro, e.g., in a cell free system, or in vivo, e.g., in a two-hybrid interaction trap assay. This method can be used to identify naturally occurring molecules that interact with subject 16836 polypeptide. It can also be used to find natural or synthetic inhibitors of subject 16836 polypeptide. Screening methods are discussed in more detail below.

### Screening Assays

The invention provides methods (also referred to herein as "screening assays") for identifying modulators, i.e., candidate or test compounds or agents (e.g., proteins, peptides, peptidomimetics, peptoids, small molecules or other drugs) which bind to 16836 proteins, have a stimulatory or inhibitory effect on, for example, 16836 expression or 16836 activity, or have a stimulatory or inhibitory effect on, for example, the expression or activity of a 16836 substrate. Compounds thus identified can be used to modulate the activity of target gene products (e.g., 16836 genes) in a therapeutic protocol, to elaborate the biological function of the target gene product, or to identify compounds that disrupt normal target gene interactions.

In one embodiment, the invention provides assays for screening candidate or test compounds which are substrates of a 16836 protein or polypeptide or a biologically active portion thereof. In another embodiment, the invention provides assays for screening candidate or test compounds that bind to or modulate an activity of a 16836 protein or polypeptide or a biologically active portion thereof.

Activities of a 16836 protein that can be evaluated in a screening assay include the ability to catalyze the hydrolysis of phosphatidylinositol, the ability to associate with ras, preferably activated (GTP-bound) ras, and the ability to mediate guanine nucleotide exchange activity.

In one embodiment, ras association activity of a 16836 protein can be assayed as follows. First a polypeptide containing a ras binding fragment of 16836, e.g., about amino acids 1640-1745 of SEQ ID NO:2, is prepared. For example, the ras binding fragment of 16836 can be fused to an amino acid sequence that allows for purification of the polypeptide and/or detection of ras binding. Second, candidate or test compounds are evaluated for their ability to modulate ras binding by the polypeptide. In one example, yeast two-hybrid screening is used to detect the interaction between a 16836 protein and ras, as well as a modulation of this interaction in the presence of candidate or test compounds. See, e.g., Shibatohge et al. (1998) J. Biol. Chem. 273 :6218 for an example of such an assay.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al. (1993) Proc. Natl. Acad Sci. U.S.A. 90:6909; Erb et al. (1994) Proc. Natl. Acad Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner, U.S. Patent No. 5,223,409), spores (Ladner U.S. Patent No. 5,223,409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad Sci. 87:6378-6382; Felici (1991) J. Mol. Biol. 222:301-310; Ladner *supra.).*

In one embodiment, an assay is a cell-based assay in which a cell which expresses a 16836 protein or biologically active portion thereof is contacted with a test compound, and the ability of the test compound to modulate 16836 activity is determined. Determining the ability of the test compound to modulate 16836 activity can be accomplished by monitoring, for example, phosphatidylinositol hydrolysis or Ras association. The cell, for example, can be of mammalian origin, e.g., human.

The ability of the test compound to modulate 16836 binding to a compound, e.g., a 16836 substrate, or to bind to 16836 can also be evaluated. This can be accomplished, for example, by coupling the compound, e.g., the substrate, with a radioisotope or enzymatic label such that binding of the compound, e.g., the substrate, to 16836 can be determined by detecting the labeled compound, e.g., substrate, in a complex. Alternatively, 16836 could be coupled with a radioisotope or enzymatic label to monitor the ability of a test compound to modulate 16836 binding to a 16836 substrate in a complex. For example, compounds (e.g., 16836 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 16836 substrate) to interact with 16836 with or without the labeling of any of the interactants can be evaluated. For example, a microphysiometer can be used to detect the interaction of a compound with 16836 without the labeling of either the compound or the 16836. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 16836.

In yet another embodiment, a cell-free assay is provided in which a 16836 protein or biologically active portion thereof is contacted with a test compound and the ability of the test compound to bind to the 16836 protein or biologically active portion thereof is evaluated. Preferred biologically active portions of the 16836 proteins to be used in assays of the present invention include fragments which participate in interactions with non-16836 molecules, e.g., fragments with high surface probability scores.

Soluble and/or membrane-bound forms of isolated proteins (e.g., 16836 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 16836 protein to bind to a target molecule can be accomplished using real-time Biomolecular Interaction Analysis (BIA) (see, e.g., Sjolander, S. and Urbaniczky, C. (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 16836, an anti-16836 antibody or its target molecule to facilitate separation of complexed from uncomplexed forms of one or both of the proteins, as well as to accommodate automation of the assay. Binding of a test compound to a 16836 protein, or interaction of a 16836 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/16836 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 16836 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 16836 binding or activity determined using standard techniques.

Other techniques for immobilizing either a 16836 protein or a target molecule on matrices include using conjugation of biotin and streptavidin. Biotinylated 16836 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 16836 protein or target molecules but which do not interfere with binding of the 16836 protein to its target molecule. Such antibodies can be derivatized to the wells of the plate, and unbound target or 16836 protein trapped in the wells by antibody conjugation. Methods for detecting such complexes, in addition to those described above for the GST-immobilized complexes, include immunodetection of complexes using antibodies reactive with the 16836 protein or target molecule, as well as enzyme-linked assays which rely on detecting an enzymatic activity associated with the 16836 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) Trends Biochem Sci 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) Current Protocols in Molecular Biology, J. Wiley: New York). Such resins and chromatographic techniques are known to one skilled in the art (see, e.g., Heegaard, N.H., (1998) J Mol Recognit 11:141-8; Hage, D.S., and Tweed, S.A. (1997) J Chromatogr B Biomed Sci Appl. 699:499-525). Further, fluorescence energy transfer may also be conveniently utilized, as described herein, to detect binding without further purification of the complex from solution.

In a preferred embodiment, the assay includes contacting the 16836 protein or biologically active portion thereof with a known compound which binds 16836 to form an assay mixture, contacting the assay mixture with a test compound, and determining the ability of the test compound to interact with a 16836 protein, wherein determining the ability of the test compound to interact with a 16836 protein includes determining the ability of the test compound to preferentially bind to 16836 or biologically active portion thereof, or to modulate the activity of a target molecule, as compared to the known compound.

The target gene products of the invention can, *in vivo*, interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 16836 genes herein identified. In an alternative embodiment, the invention provides methods for determining the ability of the test compound to modulate the activity of a 16836 protein through modulation of the activity of a downstream effector of a 16836 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U. S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 16836 proteins can be used as "bait proteins" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with 16836 ("16836-binding proteins" or "16836-bp") and are involved in 16836 activity. Such 16836-bps can be activators or inhibitors of signals by the 16836 proteins or 16836 targets as, for example, downstream elements of a 16836-mediated signaling pathway.

The two-hybrid system is based on the modular nature of most transcription factors, which consist of separable DNA-binding and activation domains. Briefly, the assay utilizes two different DNA constructs. In one construct, the gene that codes for a 16836 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 16836 protein can be the fused to the activator domain.) If the "bait" and the "prey" proteins are able to interact, *in vivo,* forming a 16836-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 16836 protein.

In another embodiment, modulators of 16836 expression are identified. For example, a cell or cell free mixture is contacted with a candidate compound and the expression of 16836 mRNA or protein evaluated relative to the level of expression of 16836 mRNA or protein in the absence of the candidate compound. When expression of 16836 mRNA or protein is greater in the presence of the candidate compound than in its absence, the candidate compound is identified as a stimulator of 16836 mRNA or protein expression. Alternatively, when expression of 16836 mRNA or protein is less (statistically significantly less) in the presence of the candidate compound than in its absence, the candidate compound is identified as an inhibitor of 16836 mRNA or protein expression. The level of 16836 mRNA or protein expression can be determined by methods described herein for detecting 16836 mRNA or protein.

In another aspect, the invention pertains to a combination of two or more of the assays described herein. For example, a modulating agent can be identified using a cell-based or a cell free assay, and the ability of the agent to modulate the activity of a 16836 protein can be confirmed *in vivo,* e.g., in an animal.

This invention further pertains to novel agents identified by the above-described screening assays. Accordingly, it is within the scope of this invention to further use an agent identified as described herein (e.g., a 16836 modulating agent, an antisense 16836 nucleic acid molecule, a 16836-specific antibody, or a 16836-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 16836 with a disease; (ii) identify an individual from a minute biological sample (tissue typing); and (iii) aid in forensic identification of a biological sample.

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 16836.

Such disorders include, e.g., a disorder associated with the misexpression of 16836 gene; a disorder of the ras-signaling system; or a disorder associated with abnormal hydrolysis of phosphatidylinositol.

The method includes one or more of the following:
detecting, in a tissue of the subject, the presence or absence of a mutation which affects the expression of the 16836 gene, or detecting the presence or absence of a mutation in a region which controls the expression of the gene, e.g., a mutation in the 5' control region;
detecting, in a tissue of the subject, the presence or absence of a mutation which alters the structure of the 16836 gene;
detecting, in a tissue of the subject, the misexpression of the 16836 gene, at the mRNA level, e.g., detecting a non-wild type level of a mRNA ;
detecting, in a tissue of the subject, the misexpression of the gene, at the protein level, e.g., detecting a non-wild type level of a 16836 polypeptide.

In preferred embodiments the method includes: ascertaining the existence of at least one of: a deletion of one or more nucleotides from the 16836 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 16836 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 16836 gene; the presence of a non-wild type splicing pattern of a messenger RNA transcript of the gene; or a non-wild type level of 16836.

Methods of the invention can be used prenatally or to determine if a subject's offspring will be at risk for a disorder.

In preferred embodiments the method includes determining the structure of a 16836 gene, an abnormal structure being indicative of risk for the disorder.

In preferred embodiments the method includes contacting a sample from the subject with an antibody to the 16836 protein or a nucleic acid, which hybridizes specifically with the gene. These and other embodiments are discussed below.

### Diagnostic and Prognostic Assays

Diagnostic and prognostic assays of the invention include method for assessing the expression level of 16836 molecules and for identifying variations and mutations in the sequence of 16836 molecules.

### Expression Monitoring and Profiling

The presence, level, or absence of 16836 protein or nucleic acid in a biological sample can be evaluated by obtaining a biological sample from a test subject and contacting the biological sample with a compound or an agent capable of detecting 16836 protein or nucleic acid (e.g., mRNA, genomic DNA) that encodes 16836 protein such that the presence of 16836 protein or nucleic acid is detected in the biological sample. The term "biological sample" includes tissues, cells and biological fluids isolated from a subject, as well as tissues, cells and fluids present within a subject. A preferred biological sample is serum. The level of expression of the 16836 gene can be measured in a number of ways, including, but not limited to: measuring the mRNA encoded by the 16836 genes; measuring the amount of protein encoded by the 16836 genes; or measuring the activity of the protein encoded by the 16836 genes.

The level of mRNA corresponding to the 16836 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 16836 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 16836 mRNA or genomic DNA The probe can be disposed on an address of an array, e.g., an array described below. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array described below. A skilled artisan can adapt known mRNA detection methods for use in detecting the level of mRNA encoded by the 16836 genes.

The level of mRNA in a sample that is encoded by one of 16836 can be evaluated with nucleic acid amplification, e.g., by rtPCR (Mullis (1987) U.S. Patent No. 4,683,202), ligase chain reaction (Barany (1991) Proc. Natl. Acad Sci. USA 88:189-193), self sustained sequence replication (Guatelli et al., (1990) Proc. Natl. Acad Sci. USA 87:1874-1878), transcriptional amplification system (Kwoh et al., (1989), Proc. Natl. Acad Sci. USA 86:1173-1177), Q-Beta Replicase (Lizardi et al., (1988) Bio/Technology 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 16836 gene being analyzed.

In another embodiment, the methods further contacting a control sample with a compound or agent capable of detecting 16836 mRNA, or genomic DNA, and comparing the presence of 16836 mRNA or genomic DNA in the control sample with the presence of 16836 mRNA or genomic DNA in the test sample. In still another embodiment, serial analysis of gene expression, as described in U.S. Patent No. 5,695,937, is used to detect 16836 transcript levels.

A variety of methods can be used to determine the level of protein encoded by 16836. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 16836 protein in a biological sample *in vitro* as well as *in vivo. In vitro* techniques for detection of 16836 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 16836 protein include introducing into a subject a labeled anti-16836 antibody. For example, the antibody can be labeled with a radioactive marker whose presence and location in a subject can be detected by standard imaging techniques. In another embodiment, the sample is labeled, e.g., biotinylated and then contacted to the antibody, e.g., an anti-16836 antibody positioned on an antibody array (as described below). The sample can be detected, e.g., with avidin coupled to a fluorescent label.

In another embodiment, the methods further include contacting the control sample with a compound or agent capable of detecting 16836 protein, and comparing the presence of 16836 protein in the control sample with the presence of 16836 protein in the test sample.

The invention also includes kits for detecting the presence of 16836 in a biological sample. For example, the kit can include a compound or agent capable of detecting 16836 protein or mRNA in a biological sample; and a standard. The compound or agent can be packaged in a suitable container. The kit can further comprise instructions for using the kit to detect 16836 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 16836 expression or activity. As used herein, the term "unwanted" includes an unwanted phenomenon involved in a biological response such as cancer or deregulated cell proliferation.

In one embodiment, a disease or disorder associated with aberrant or unwanted 16836 expression or activity is identified. A test sample is obtained from a subject and 16836 protein or nucleic acid (e.g., mRNA or genomic DNA) is evaluated, wherein the level, e.g., the presence or absence, of 16836 protein or nucleic acid is diagnostic for a subject having or at risk of developing a disease or disorder associated with aberrant or unwanted 16836 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 16836 expression or activity. For example, such methods can be used to determine whether a subject can be effectively treated with an agent for a cellular proliferation or differentiation related disorder.

In another aspect, the invention features a computer medium having a plurality of digitally encoded data records. Each data record includes a value representing the level of expression of 16836 in a sample, and a descriptor of the sample. The descriptor of the sample can be an identifier of the sample, a subject from which the sample was derived (e.g., a patient), a diagnosis, or a treatment (e.g., a preferred treatment). In a preferred embodiment, the data record further includes values representing the level of expression of genes other than 16836 (e.g., other genes associated with a 16836-disorder, or other genes on an array). The data record can be structured as a table, e.g., a table that is part of a database such as a relational database (e.g., a SQL database of the Oracle or Sybase database environments).

Also featured is a method of evaluating a sample. The method includes providing a sample, e.g., from the subject, and determining a gene expression profile of the sample, wherein the profile includes a value representing the level of 16836 expression. The method can further include comparing the value or the profile (i.e., multiple values) to a reference value or reference profile. The gene expression profile of the sample can be obtained by any of the methods described herein (e.g., by providing a nucleic acid from the sample and contacting the nucleic acid to an array). The method can be used to diagnose a cellular proliferative and/or differentiative disorder in a subject wherein an increase in 16836 expression is an indication that the subject has or is disposed to having a cellular proliferative and/or differentiative disorder. The method can be used to monitor a treatment for cellular proliferative and/or differentiative disorder in a subject. For example, the gene expression profile can be determined for a sample from a subject undergoing treatment. The profile can be compared to a reference profile or to a profile obtained from the subject prior to treatment or prior to onset of the disorder (see, e.g., Golub et al. (1999) Science 286:531).

In yet another aspect, the invention features a method of evaluating a test compound (see also, "Screening Assays", above). The method includes providing a cell and a test compound; contacting the test compound to the cell; obtaining a subject expression profile for the contacted cell; and comparing the subject expression profile to one or more reference profiles. The profiles include a value representing the level of 16836 expression. In a preferred embodiment, the subject expression profile is compared to a target profile, e.g., a profile for a normal cell or for desired condition of a cell. The test compound is evaluated favorably if the subject expression profile is more similar to the target profile than an expression profile obtained from an uncontacted cell.

In another aspect, the invention features, a method of evaluating a subject. The method includes: a) obtaining a sample from a subject, e.g., from a caregiver, e.g., a caregiver who obtains the sample from the subject; b) determining a subject expression profile for the sample. Optionally, the method further includes either or both of steps: c) comparing the subject expression profile to one or more reference expression profiles; and d) selecting the reference profile most similar to the subject reference profile. The subject expression profile and the reference profiles include a value representing the level of 16836 expression. A variety of routine statistical measures can be used to compare two reference profiles. One possible metric is the length of the distance vector that is the difference between the two profiles. Each of the subject and reference profile is represented as a multi-dimensional vector, wherein each dimension is a value in the profile.

The method can further include transmitting a result to a caregiver. The result can be the subject expression profile, a result of a comparison of the subject expression profile with another profile, a most similar reference profile, or a descriptor of any of the aforementioned. The result can be transmitted across a computer network, e.g., the result can be in the form of a computer transmission, e.g., a computer data signal embedded in a carrier wave.

Also featured is a computer medium having executable code for effecting the following steps: receive a subject expression profile; access a database of reference expression profiles; and either i) select a matching reference profile most similar to the subject expression profile or ii) determine at least one comparison score for the similarity of the subject expression profile to at least one reference profile. The subject expression profile, and the reference expression profiles each include a value representing the level of 16836 expression.

### Pharmaceutical Compositions

The nucleic acid and polypeptides, fragments thereof, as well as anti-16836 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193*).*

The present invention encompasses agents which modulate expression or activity. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al. (1994) Proc. Natl. Acad Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Methods of Treatment

The present invention provides for both prophylactic and therapeutic methods of treating a subject at risk of (or susceptible to) a disorder or having a disorder associated with aberrant or unwanted 16836 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 16836 molecules of the present invention or 16836 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 16836 expression or activity, by administering to the subject a 16836 or an agent which modulates 16836 expression or at least one 16836 activity. Subjects at risk for a disease which is caused or contributed to by aberrant or unwanted 16836 expression or activity can be identified by, for example, any or a combination of diagnostic or prognostic assays as described herein. Administration of a prophylactic agent can occur prior to the manifestation of symptoms characteristic of the 16836 aberrance, such that a disease or disorder is prevented or, alternatively, delayed in its progression. Depending on the type of 16836 aberrance, for example, a 16836, 16836 agonist or 16836 antagonist agent can be used for treating the subject. The appropriate agent can be determined based on screening assays described herein.

It is possible that some 16836 disorders can be caused, at least in part, by an abnormal level of gene product, or by the presence of a gene product exhibiting abnormal activity. As such, the reduction in the level and/or activity of such gene products would bring about the amelioration of disorder symptoms.

As shown in Figure 4, expression of 16836 mRNA is detected in bone cells (e.g., osteoclasts), testis, brain, skeletal muscle, breast, heart, and fetal liver. Accordingly, the molecules of the invention may mediate disorders involving aberrant activities of these cells, e.g., bone disorders, brain disorders, breast, reproductive disorders (e.g., testiculat disorders), cardiovascular disorders, skeletal muscle disorders, or immune disorders.

As discussed, successful treatment of 16836 disorders can be brought about by techniques that serve to inhibit the expression or activity of target gene products. For example, compounds, e.g., an agent identified using an assays described above, that proves to exhibit negative modulatory activity, can be used in accordance with the invention to prevent and/or ameliorate symptoms of 16836 disorders. Such molecules can include, but are not limited to peptides, phosphopeptides, small organic or inorganic molecules, or antibodies (including, for example, polyclonal, monoclonal, humanized, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules may be utilized in treating or preventing a disease characterized by 16836 expression is through the use of aptamer molecules specific for 16836 protein. Aptamers are nucleic acid molecules having a tertiary structure which permits them to specifically bind to protein ligands (see, e.g., Osborne, et al. (1997) Curr. Opin. Chem Biol. 1: 5-9; and Patel, D.J. (1997) Curr Opin Chem Biol 1:32-46). Since nucleic acid molecules may in many cases be more conveniently introduced into target cells than therapeutic protein molecules may be, aptamers offer a method by which 16836 protein activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of 16836 disorders. For a description of antibodies, see the Antibody section above.

In circumstances wherein injection of an animal or a human subject with a 16836 protein or epitope for stimulating antibody production is harmful to the subject, it is possible to generate an immune response against 16836 through the use of anti-idiotypic antibodies (see, for example, Herlyn, D. (1999) Ann Med 31:66-78; and Bhattacharya-Chatterjee, M., and Foon, K.A. (1998) Cancer Treat Res. 94:51-68). If an anti-idiotypic antibody is introduced into a mammal or human subject, it should stimulate the production of anti-anti-idiotypic antibodies, which should be specific to the 16836 protein. Vaccines directed to a disease characterized by 16836 expression may also be generated in this fashion.

In instances where the target antigen is intracellular and whole antibodies are used, internalizing antibodies may be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco et al. (1993) Proc. Natl. Acad Sci. USA 90:7889-7893).

The identified compounds that inhibit target gene expression, synthesis and/or activity can be administered to a patient at therapeutically effective doses to prevent, treat or ameliorate 16836 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is the ability to directly assay levels of "free" and "bound" compound in the serum of the test subject. Such assays may utilize antibody mimics and/or "biosensors" that have been created through molecular imprinting techniques. The compound which is able to modulate 16836 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell, R J. et al (1996) Current Opinion in Biotechnology 7:89-94 and in Shea, K.J. (1994) Trends in Polymer Science 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, G. et al (1993) Nature 361:645-647. Through the use of isotope-labeling, the "free" concentration of compound which modulates the expression or activity of 16836 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. An rudimentary example of such a "biosensor" is discussed in Kriz, D. et al (1995) Analytical Chemistry 67:2142-2144.

Another aspect of the invention pertains to methods of modulating 16836 expression or activity for therapeutic purposes. Accordingly, in an exemplary embodiment, the modulatory method of the invention involves contacting a cell with a 16836 or agent that modulates one or more of the activities of 16836 protein activity associated with the cell. An agent that modulates 16836 protein activity can be an agent as described herein, such as a nucleic acid or a protein, a naturally-occurring target molecule of a 16836 protein (e.g., a 16836 substrate or receptor), a 16836 antibody, a 16836 agonist or antagonist, a peptidomimetic of a 16836 agonist or antagonist, or other small molecule.

In one embodiment, the agent stimulates one or 16836 activities. Examples of such stimulatory agents include active 16836 protein and a nucleic acid molecule encoding 16836. In another embodiment, the agent inhibits one or more 16836 activities. Examples of such inhibitory agents include antisense 16836 nucleic acid molecules, anti-16836 antibodies, and 16836 inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder characterized by aberrant or unwanted expression or activity of a 16836 protein or nucleic acid molecule. In one embodiment, the method involves administering an agent (e.g., an agent identified by a screening assay described herein), or combination of agents that modulates (e.g., up regulates or down regulates) 16836 expression or activity. In another embodiment, the method involves administering a 16836 protein or nucleic acid molecule as therapy to compensate for reduced, aberrant, or unwanted 16836 expression or activity.

Stimulation of 16836 activity is desirable in situations in which 16836 is abnormally downregulated and/or in which increased 16836 activity is likely to have a beneficial effect. For example, stimulation of 16836 activity is desirable in situations in which a 16836 is downregulated and/or in which increased 16836 activity is likely to have a beneficial effect. Likewise, inhibition of 16836 activity is desirable in situations in which 16836 is abnormally upregulated and/or in which decreased 16836 activity is likely to have a beneficial effect.

### Pharmacogenomics

The 16836 molecules of the present invention, as well as agents, or modulators which have a stimulatory or inhibitory effect on 16836 activity (e.g., 16836 gene expression) as identified by a screening assay described herein can be administered to individuals to treat (prophylactically or therapeutically) 16836 associated disorders (e.g., cellular proliferative and/or differentiative disorders) associated with aberrant or unwanted 16836 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 16836 molecule or 16836 modulator as well as tailoring the dosage and/or therapeutic regimen of treatment with a 16836 molecule or 16836 modulator.

Pharmacogenomics deals with clinically significant hereditary variations in the response to drugs due to altered drug disposition and abnormal action in affected persons. See, for example, Eichelbaum, M. et al. (1996) Clin. Exp. Pharmacol. Physiol. 23:983-985 and Linder, M.W. et al. (1997) Clin. Chem. 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach," can be utilized to identify genes that predict drug response. According to this method, if a gene that encodes a drug's target is known (e.g., a 16836 protein of the present invention), all common variants of that gene can be fairly easily identified in the population and it can be determined if having one version of the gene versus another is associated with a particular drug response.

Alternatively, a method termed the "gene expression profiling," can be utilized to identify genes that predict drug response. For example, the gene expression of an animal dosed with a drug (e.g., a 16836 molecule or 16836 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 16836 molecule or 16836 modulator, such as a modulator identified by one of the exemplary screening assays described herein.

The present invention further provides methods for identifying new agents, or combinations, that are based on identifying agents that modulate the activity of one or more of the gene products encoded by one or more of the 16836 genes of the present invention, wherein these products may be associated with resistance of the cells to a therapeutic agent. Specifically, the activity of the proteins encoded by the 16836 genes of the present invention can be used as a basis for identifying agents for overcoming agent resistance. By blocking the activity of one or more of the resistance proteins, target cells, e.g., human cells, will become sensitive to treatment with an agent that the unmodified target cells were resistant to.

Monitoring the influence of agents (e.g., drugs) on the expression or activity of a 16836 protein can be applied in clinical trials. For example, the effectiveness of an agent determined by a screening assay as described herein to increase 16836 gene expression, protein levels, or upregulate 16836 activity, can be monitored in clinical trials of subjects exhibiting decreased 16836 gene expression, protein levels, or downregulated 16836 activity. Alternatively, the effectiveness of an agent determined by a screening assay to decrease 16836 gene expression, protein levels, or downregulate 16836 activity, can be monitored in clinical trials of subjects exhibiting increased 16836 gene expression, protein levels, or upregulated 16836 activity. In such clinical trials, the expression or activity of a 16836 gene, and preferably, other genes that have been implicated in, for example, a 16836-associated disorder can be used as a "read out" or markers of the phenotype of a particular cell.

### EXAMPLES

### Example 1: Identification and Characterization of Human 16836 cDNA

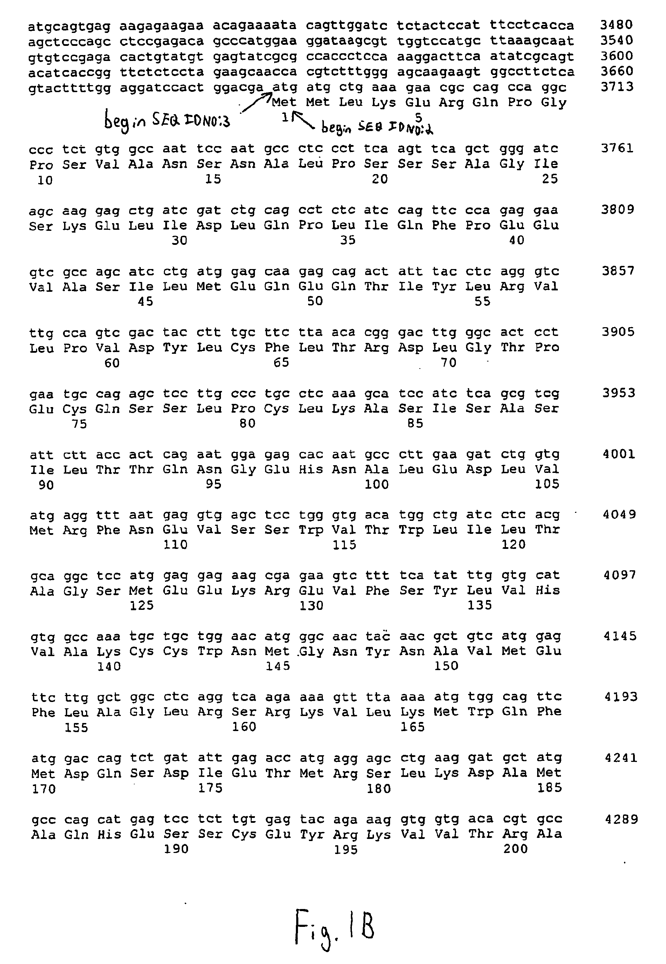

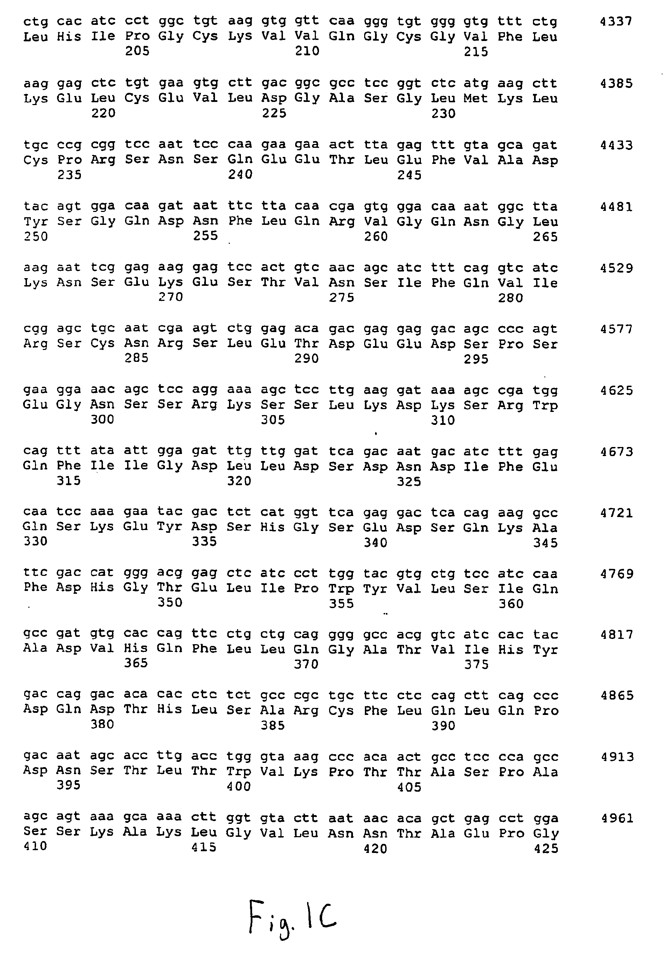

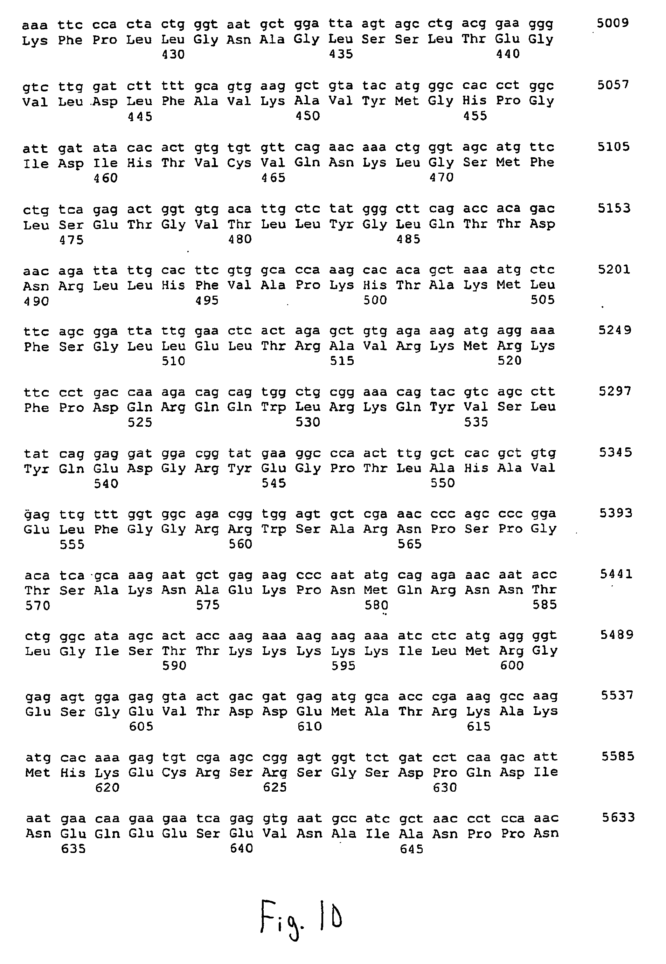

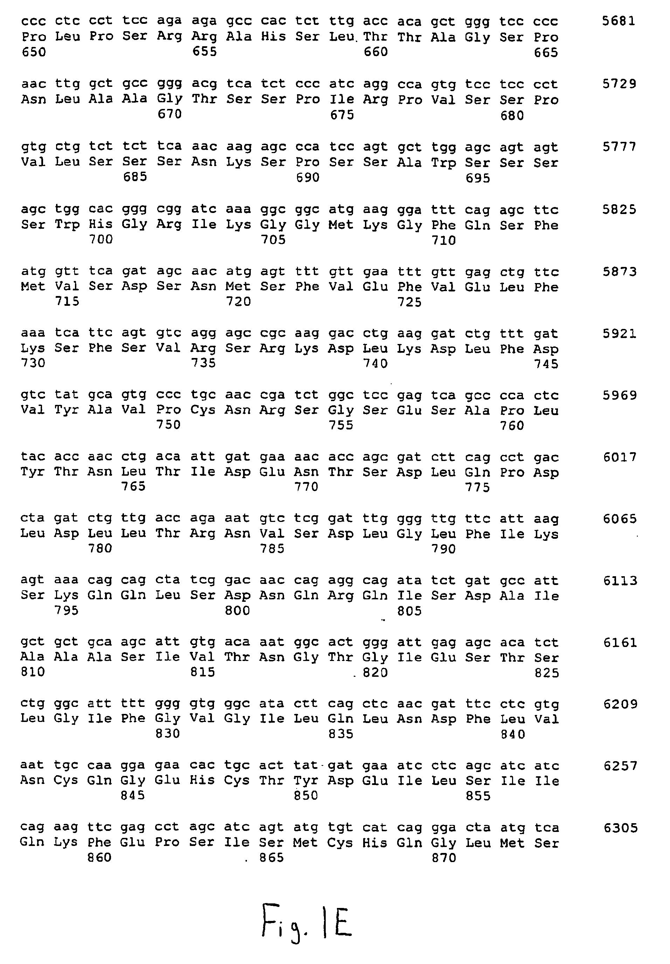

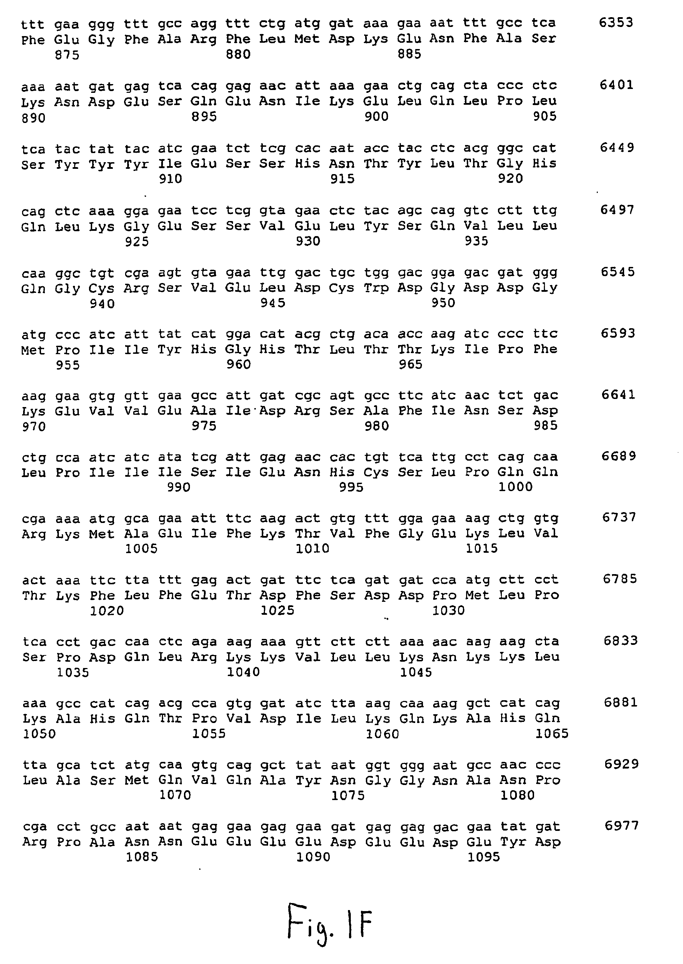

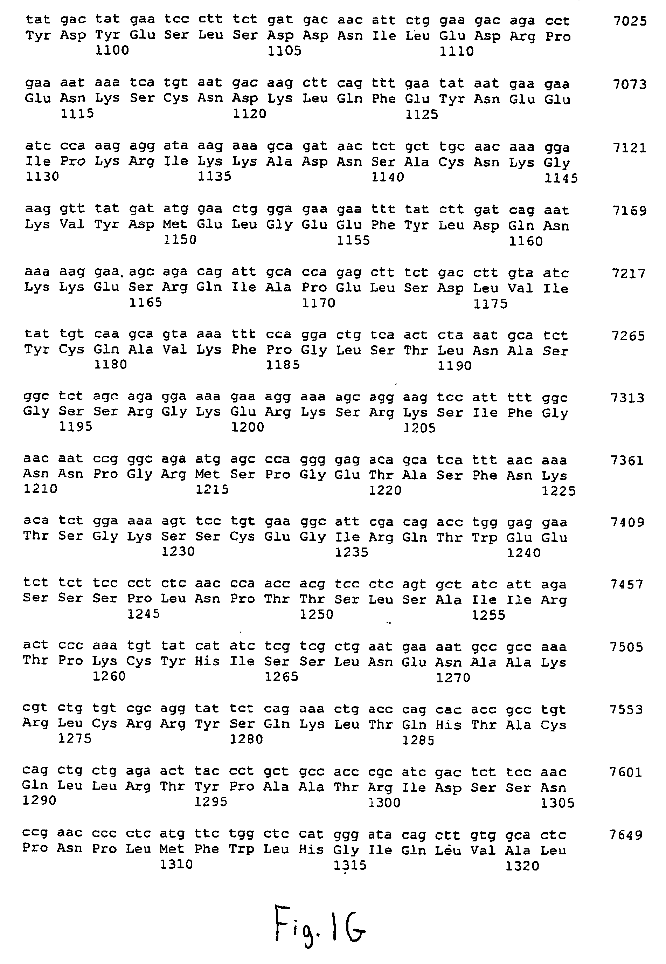

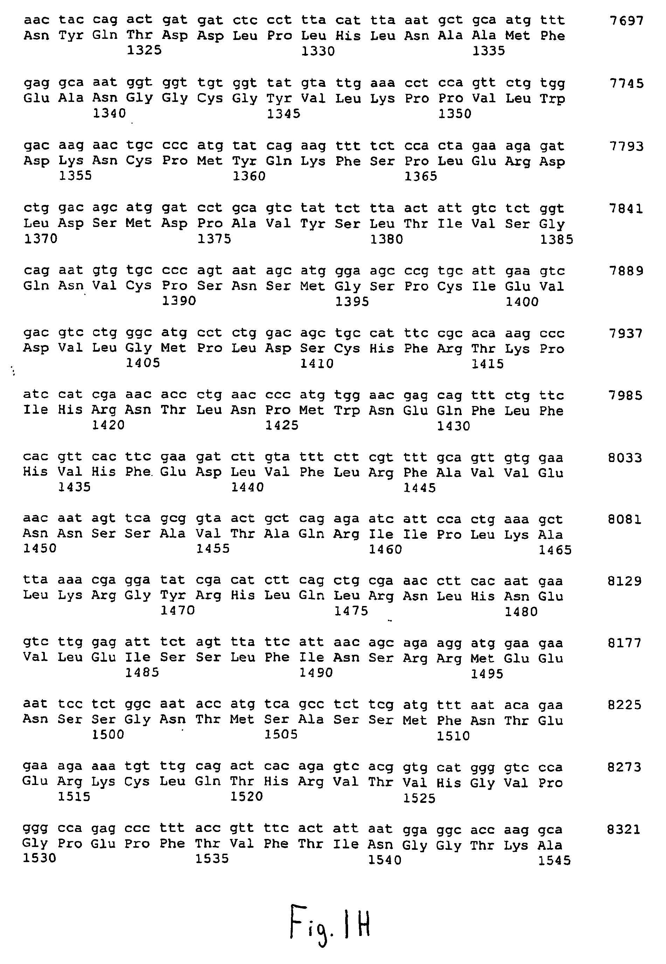

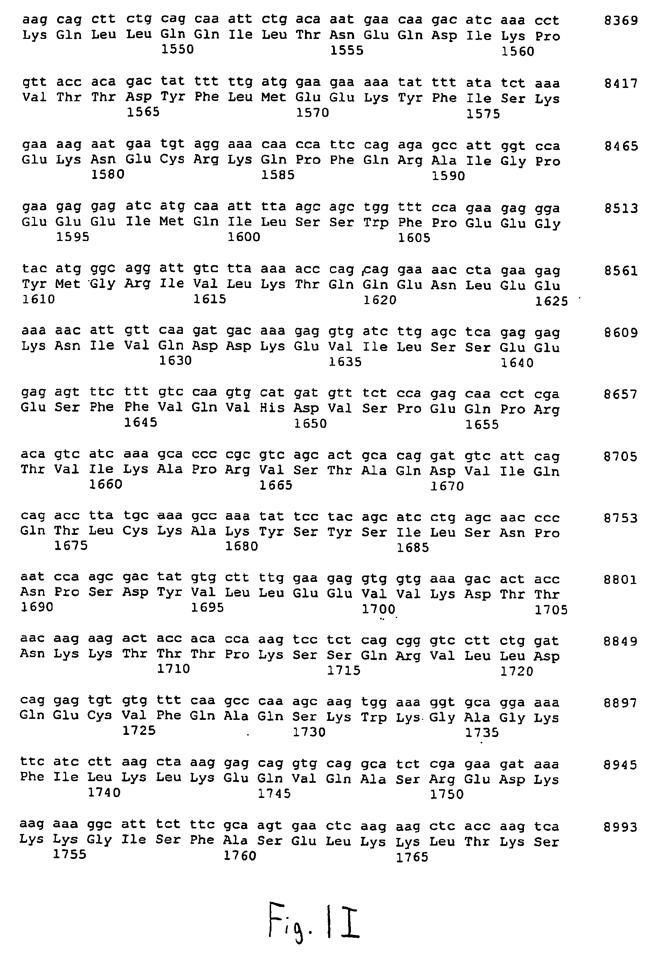

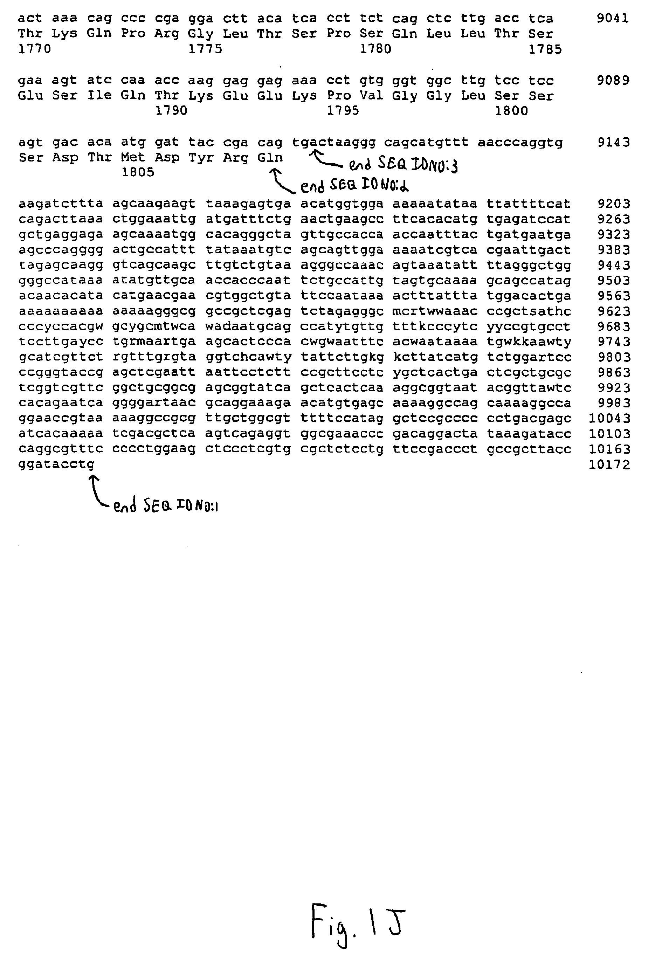

The human 16836 sequence (Figs. 1A-1J; SEQ ID NO:1), which is approximately 10,172 nucleotides long, including untranslated regions, contains a predicted methionine-initiated coding sequence of about 5,430 nucleotides, including the termination codon (nucleotides indicated as "coding" of SEQ ID NO:1 in Fig. 1; SEQ ID NO:3). The coding sequence encodes a 1809 amino acid protein (SEQ ID NO:2).

### Example 2: Tissue Distribution of 16836 mRNA by TaqMan Analysis

Endogenous human 16836 gene expression was determined using the Perkin-Elmer/ABI 7700 Sequence Detection System which employs TaqMan technology. Briefly, TaqMan technology relies on standard RT-PCR with the addition of a third gene-specific oligonucleotide (referred to as a probe) which has a fluorescent dye coupled to its 5' end (typically 6-FAM) and a quenching dye at the 3' end (typically TAMRA). When the fluorescently tagged oligonucleotide is intact, the fluorescent signal from the 5' dye is quenched. As PCR proceeds, the 5' to 3' nucleolytic activity oftaq polymerase digests the labeled primer, producing a free nucleotide labeled with 6-FAM, which is now detected as a fluorescent signal. The PCR cycle where fluorescence is first released and detected is directly proportional to the starting amount of the gene of interest in the test sample, thus providing a way of quantitating the initial template concentration. Samples can be internally controlled by the addition of a second set of primers/probe specific for a housekeeping gene such as GAPDH which has been labeled with a different fluorophore on the 5' end (typically VIC).

To determine the level of 16836 in various human tissues a primer/probe set was designed using Primer Express (Perkin-Elmer) software and primary cDNA sequence information. Total RNA was prepared from a series of human tissues using an RNeasy kit from Qiagen. First strand cDNA was prepared from one ug total RNA using an oligo dT primer and Superscript II reverse transcriptase (GibcoBRL). cDNA obtained from approximately 50 ng total RNA was used per TaqMan reaction.

Normal tissues tested included the human tissues provided in Figure 4, including bone cells (e.g., osteoclasts and osteoblasts), liver, fetal liver, brain, trachea, skeletal muscle, heart, thyroid, skin, testis, breast, and placenta, among others. Expression was found primarily in osteoclasts, testis, brain, skeletal muscle, breast, heart and fetal liver (Figure 4).

Increased expression of 16836 was seen in lung tumors ("lung T"; small cell carcinoma (SCC) and adenocarcinoma (AC)) when compared to normal (N) lung tissue (Figure 5). Association of elevated ex-pression of 16836 with activating mutations of kras was found in both lung tumor samples (Figure 6A) and tumor cell lines derived from the breast, colon, and lung (Figure 6B).

In a panel comprising normal (N) and tumor (T) tissues from the breast, lung, colon, and liver, increased expression of 16836 was seen in the tumor tissues, especially tumors of the breast, lung, and colon (Figure 7). An analysis of 16836 expression in tumor as compared to normal tissues of the breast is depicted in Figure 8. An analysis of 16836 expression in several transformed cell lines is depicted in Figure 9. These cell lines included human breast cancer cell lines (MCF-7, ZR75, T47D, and MDA), human colon cancer cell lines (DLD-1, SW480, SW620, HCT116, HT 29, and Colo205), and human lung cancer cell lines (NCI-H125 and A549).

The incidence of tumor associated expression of 16836 in tumors of the colon, breast, and lung was evaluated by *in situ* hybridization. Moderate expression of 16836 was detected in colonic tumor cells (expression in 0/2 normal samples; expression in 2/3 tumor samples; and expression in 0/2 metastases samples). Slightly positive expression was seen focally in normal epithelium in one breast tumor sample (expression in 0/2 normal samples; and expression in normal epithelium in 1/4 tumor samples). Moderate expression was seen in inflammatory cells of lung tumor samples (expression in 0/2 normal samples; and expression in 2/4 tumor samples).

### Example 3: Tissue Distribution of 16836 mRNA by Northern Analysis

Northern blot hybridizations with various RNA samples can be performed under standard conditions and washed under stringent conditions, i.e., 0.2xSSC at 65°C. A DNA probe corresponding to all or a portion of the 16836 cDNA (SEQ ID NO:1) can be used. The DNA was radioactively labeled with ³²P-dCTP using the Prime-It Kit (Stratagene, La Jolla, CA) according to the instructions of the supplier. Filters containing mRNA from mouse hematopoietic and endocrine tissues, and cancer cell lines (Clontech, Palo Alto, CA) can be probed in ExpressHyb hybridization solution (Clontech) and washed at high stringency according to manufacturer's recommendations.

Northern blot hybridizations were performed using a labeled 16836 probe and RNA samples from various lung tumors and tumor cell lines. A 16836 transcript greater than 7.5 kb in size was detected in some of these samples. The results were as follows: NHBE (negative); A549 (very strong positive); NCI-H69 (negative); NCI-H125 (strong positive); NCI-H322 (positive); and NCI-H460 (positive).

### Example 4: Recombinant Expression of 16836 in Bacterial Cells

In this example, 16836 is expressed as a recombinant glutathione-S-transferase (GST) fusion polypeptide in *E. coli* and the fusion polypeptide is isolated and characterized. Specifically, 16836 is fused to GST and this fusion polypeptide is expressed in *E. coli,* e.g., strain PEB199. Expression of the GST-16836 fusion protein in PEB199 is induced with IPTG. The recombinant fusion polypeptide is purified from crude bacterial lysates of the induced PEB 199 strain by affinity chromatography on glutathione beads. Using polyacrylamide gel electrophoretic analysis of the polypeptide purified from the bacterial lysates, the molecular weight of the resultant fusion polypeptide is determined.

### Example 5: Expression of Recombinant 16836 Protein in COS Cells

To express the 16836 gene in COS cells, the pcDNA/Amp vector by Invitrogen Corporation (San Diego, CA) is used. This vector contains an SV40 origin of replication, an ampicillin resistance gene, an *E. coli* replication origin, a CMV promoter followed by a polylinker region, and an SV40 intron and polyadenylation site. A DNA fragment encoding the entire 16836 protein and an HA tag (Wilson et al. (1984) Cell 37:767) or a FLAG tag fused in-frame to its 3' end of the fragment is cloned into the polylinker region of the vector, thereby placing the expression of the recombinant protein under the control of the CMV promoter.

To construct the plasmid, the 16836 DNA sequence is amplified by PCR using two primers. The 5' primer contains the restriction site of interest followed by approximately twenty nucleotides of the 16836 coding sequence starting from the initiation codon; the 3' end sequence contains complementary sequences to the other restriction site of interest, a translation stop codon, the HA tag or FLAG tag and the last 20 nucleotides of the 16836 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 16836_gene is inserted in the correct orientation. The ligation mixture is transformed into *E*. *coli* cells (strains HB101, DH5α, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the 16836-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. (1989) Molecular Cloning: A Laboratory Manual. 2nd, ed., Cold Spring Harbor Laboratory, Cold Spring Harbor Laboratory Press, Cold Spring Harbor, NY. The expression of the 16836 polypeptide is detected by radiolabelling (³⁵S-methionine or ³⁵S-cysteine available from NEN, Boston, MA, can be used) and immunoprecipitation (Harlow, E. and Lane, D. (1988) Antibodies: A Laboratory Manual, Cold Spring Harbor Laboratory Press, Cold Spring Harbor, NY) using an HA specific monoclonal antibody. Briefly, the cells are labeled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 16836 coding sequence is cloned directly into the polylinker of the pCDNA/Amp vector using the appropriate restriction sites. The resulting plasmid is transfected into COS cells in the manner described above, and the expression of the 16836 polypeptide is detected by radiolabelling and immunoprecipitation using a 16836 specific monoclonal antibody.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a) a nucleic acid molecule consisting of a nucleotide sequence which is at least 80% identical to the entire length of the nucleotide sequence of SEQ ID NO:1, or at least 92% identical to the entire length of the nucleotide sequence of SEQ ID NO:3;
b) a nucleic acid molecule which encodes a polypeptide comprising the amino acid sequence of SEQ ID NO:2; and
c) a nucleic acid molecule which encodes a fragment of a polypeptide comprising the amino acid sequence of SEQ ID NO:2, wherein the fragment comprises at least 1200 contiguous amino acids of SEQ ID NO: 2.

2. The isolated nucleic acid molecule of claim 1, which is selected from the group consisting of:
a) a nucleic acid comprising the nucleotide sequence of SEQ ID NO: 1, SEQ ID NO:3; and
b) a nucleic acid molecule which encodes a polypeptide comprising the amino acid sequence of SEQ ID NO:2.

3. The nucleic acid molecule of claim 1 further comprising vector nucleic acid sequences.

4. The nucleic acid molecule of claim 1 further comprising nucleic acid sequences encoding a heterologous polypeptide.

5. A host cell which contains the nucleic acid molecule of claim 1.

6. The host cell of claim 5 which is a mammalian host cell.

7. A non-human mammalian host cell containing the nucleic acid molecule of claim 1.

8. An isolated polypeptide selected from the group consisting of:
a) a polypeptide which is encoded by a nucleic acid molecule consisting of a nucleotide sequence which is at least 80% identical to the entire length of the nucleotide sequence of SEQ ID NO: 1 or at least 92% identical to the entire length of the nucleotide sequence of SEQ ID NO:3; and
b) a fragment of a polypeptide comprising the amino acid sequence of SEQ ID NO:2, wherein the fragment comprises at least 1200 contiguous amino acids of SEQ ID NO:2.

9. The isolated polypeptide of claim 8 comprising the amino acid sequence of SEQ ID NO:2.

10. The polypeptide of claim 8 further comprising heterologous amino acid sequences.

11. An antibody or immunologically active portion thereof, which selectively binds to a polypeptide selected from the group consisting of:
a) a polypeptide which is encoded by a nucleic acid molecule consisting of a nucleotide sequence which is at least 80% identical to the entire length of the nucleotide sequence of SEQ ID NO: 1 or at least 92% identical to the entire length of the nucleotide sequence of SEQ ID NO:3; and
b) a fragment of a polypeptide consisting of the amino acid sequence of SEQ ID NO:2, wherein the fragment comprises at least 1200 contiguous amino acids of SEQ ID NO:2.

12. The antibody or immunologically active portion thereof of claim 11, wherein the antibody is detectably labeled.

13. The antibody or immunologically active portion thereof of claim 12, wherein the detectable label is selected from the group consisting of:
a) enzymes;
b) prosthetic groups;
c) fluorescent materials;
d) luminescent materials;
e) bioluminescent materials; and
f) radioactive materials.

14. The antibody or immunologically active portion thereof of claim 11, wherein the antibody is selected from the group consisting of:
a) monoclonal antibody;
b) polyclonal antibody;
c) Fab fragment;
d) F(ab')₂ fragment;
e) chimeric antibody;
f) humanized antibody; and
g) human antibody

15. A method for producing a polypeptide selected from the group consisting of:
a) a polypeptide comprising the amino acid sequence of SEQ ID NO:2; and
b) a polypeptide comprising a fragment of the amino acid sequence of SEQ ID NO:2, wherein the fragment comprises at least 1200 contiguous amino acids of SEQ ID NO:2;
the method comprising culturing the host cell of claim 5 under conditions in which the nucleic acid molecule is expressed.

16. A method for detecting the presence of a polypeptide of claim 8 in a sample, comprising:
a) contacting the sample with a compound which selectively binds to a polypeptide of claim 8; and
b) determining whether the compound binds to the polypeptide in the sample.

17. The method of claim 16, wherein the compound which binds to the polypeptide is an antibody.

18. A kit comprising an antibody which selectively binds to a polypeptide of claim 8 and instructions for use.

19. A method for detecting the presence of a nucleic acid molecule of claim 1 in a sample, comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

20. The method of claim 19, wherein the sample comprises mRNA molecules and is contacted with a nucleic acid probe.

21. A kit comprising a nucleic acid probe or primer which selectively hybridizes to a nucleic acid molecule of claim 1 and instructions for use.

22. A method for identifying a compound which binds to a polypeptide of claim 8 comprising the steps of:
a) contacting a polypeptide, or a cell expressing a polypeptide of claim 8 with a test compound; and
b) determining whether the polypeptide binds to the test compound.

23. The method of claim 22, wherein the binding of the test compound to the polypeptide is detected by a method selected from the group consisting of:
a) detection of binding by direct detecting of test compound/polypeptide binding; and
b) detection of binding using a competition binding assay.

24. A method for modulating the activity of a polypeptide of claim 8 comprising contacting a polypeptide or a cell expressing a polypeptide of claim 8 with an antibody which binds to the polypeptide in a sufficient concentration to modulate the activity of the polypeptide.

25. A method for identifying an agent which modulates the activity of a polypeptide of claim 8, comprising:
a) contacting a polypeptide of claim 8 with a test agent; and
b) determining the effect of the test compound on the activity of the polypeptide to thereby identify an agent which modulates the activity of the polypeptide.

26. Use of an agent that modulates the activity or expression of a nucleic acid of claim 1 or polypeptide of claim 8 in the manufacture of a medicament for the treatment or prevention of a cellular proliferative or differentiative disorder **characterized by** aberrant activity or expression of a nucleic acid of claim 1 or polypeptide of claim 8 in a subject, the treatment comprising administering to the subject an effective amount of the agent that modulates the activity or expression of a nucleic acid of claim 1 or polypeptide of claim 8 such that the cellular proliferative or differentiative disorder is ameliorated or prevented, wherein the agent is selected from the group consisting of a polypeptide of claim 8, a nucleic acid molecule of claim 1, an antibody, a ribozyme, and an antisense molecule.

27. The use of claim 26, wherein the cellular proliferative or differentiative disorder is lung cancer, colon cancer, or breast cancer.

28. An in vitro method for identifying an agent that modulates the activity or expression of a polypeptide of claim 8 or a nucleic acid molecule of claim 1, the method comprising:
a) contacting the polypeptide or nucleic acid with an agent; and
b) determining the effect of the agent on the activity or expression of the polypeptide or nucleic acid; wherein the agent is selected from the group consisting of a polypeptide of claim 8, a nucleic acid molecule of claim 1, an antibody, a ribozyme, and an antisense molecule.

29. The method of claim 28, which comprises contacting a polypeptide of claim 8 with the agent and determining the effect of the agent on the ability of the polypeptide to catalyze the hydrolysis of phosphatidylinositol, or which comprises contacting a polypeptide of claim 8 with the agent and determining the effect of the agent on the ability of the polypeptide to associate with a Ras protein, or which comprises contacting a polypeptide of claim 8 with the agent and determining the effect of the agent on the ability of the polypeptide to mediate guanine nucleotide exchange activity.

30. An in vitro method of modulating the activity of a cell expressing a polypeptide of claim 8 or a nucleic acid molecule of claim 1, comprising contacting the cell with an amount of an agent that modulates the activity or expression of a nucleic acid of claim 1 or a polypeptide of claim 8 such that the activity of the cell is modulated; wherein the agent is selected from the group consisting of a polypeptide of claim 8, a nucleic acid molecule of claim 1, an antibody, a ribozyme, and an antisense molecule.

## Patentansprüche

1. Isoliertes Nukleinsäuremolekül ausgewählt aus der Gruppe bestehend aus:
a) einem Nukleinsäuremolekül bestehend aus einer Sequenz, die zumindest 80 % identisch zu der gesamten Länge der Nukleinsäuresequenz aus SEQ ID NO: 1 oder zumindest 92 % identisch zu der gesamten Länge der Nukleinsäuresequenz aus SEQ ID NO: 3 ist;
b) einem Nukleinsäuremolekül, das für ein Polypeptid kodiert, welches die Aminosäuresequenz nach SEQ ID NO: 2 umfasst; und
c) einem Nukleinsäuremolekül, das für ein Fragment eines Polypeptids kodiert, welches die Aminosäuresequenz nach SEQ ID NO: 2 umfasst, wobei das Fragment zumindest 1200 zusammenhängende Aminosäuren aus SEQ ID NO: 2 umfasst.

2. Das isolierte Nukleinsäuremolekül gemäß Anspruch 1, das ausgewählt ist aus der Gruppe bestehend aus:
a) einer Nukleinsäure, welche die Nukleinsäuresequenz nach SEQ ID NO: 1, SEQ ID NO: 3 umfasst; und
b) einem Nukleinsäuremolekül, das für ein Polypeptid kodiert, welches die Aminosäuresequenz nach SEQ ID NO: 2 umfasst.

3. Das Nukleinsäuremolekül gemäß Anspruch 1, weiterhin umfassend Nukleinsäuresequenzen eines Vektors.

4. Das Nukleinsäuremolekül gemäß Anspruch 1, weiterhin umfassend Nukleinsäuresecluenzen, die für ein heterologes Polypeptid kodieren.

5. Eine Wirtszelle, die das Nukleinsäuremolekül nach Anspruch 1 enthält.

6. Die Wirtszelle nach Anspruch 5, die eine Säugetier-Wirtszelle ist.

7. Eine nicht-menschliche Säugetier-Wirtszelle enthaltend das Nukleinsäuremolekül nach Anspruch 1.

8. Ein isoliertes Polypeptid ausgewählt aus der Gruppe bestehend aus:
a) einem Polypeptid, für welches ein Nukleinsäuremolekül kodiert, das aus einer Nukleotidsequenz besteht, die zumindest 80 % identisch ist zu der gesamten Länge der Nukleinsäuresequenz nach SEQ ID NO: 1 oder zumindest 92 % identisch zu der gesamten Länge der Nukleinsäuresequenz nach SEQ ID NO: 3 ist; und
b) einem Fragment eines Polypeptids, das die Aminosäuresequenz nach SEQ ID NO: 2 umfasst, wobei das Fragment zumindest 1200 zusammenhängende Aminosäuresequenzen aus SEQ ID NO: 2 umfasst.

9. Das isolierte Polypeptid nach Anspruch 8, umfassend die Aminosäuresequenz nach SEQ ID NO: 2.

10. Das Polypeptid nach Anspruch 8, weiterhin umfassend heterologe Aminosäuresequenzen.

11. Ein Antikörper oder ein immunologisch aktiver Teil davon, welcher selektiv an ein Polypeptid bindet, das ausgewählt ist aus der Gruppe bestehend aus:
a) einem Polypeptid, für welches ein Nukleinsäuremolekül kodiert, das aus einer Nukleotidsequenz besteht, die zumindest 80 % identisch ist zu der gesamten Länge der Nukleinsäuresequenz nach SEQ ID NO: 1 oder zumindest 92 % identisch zu der gesamten Länge der Nukleinsäuresequenz nach SEQ ID NO: 3 ist; und
b) einem Fragment eines Polypeptids, das die Aminosäuresequenz nach SEQ ID NO: 2 umfasst, wobei das Fragment zumindest 1200 zusammenhängende Aminosäuresequenzen aus SEQ ID NO: 2 umfasst.

12. Der Antikörper oder der immunologisch aktive Teil davon gemäß Anspruch 11, wobei der Antikörper detektierbar markiert ist.

13. Der Antikörper oder der immunologisch aktive Teil davon gemäß Anspruch 12, wobei die detektierbare Markierung ausgewählt ist aus der Gruppe bestehend aus:
a) Enzymen;
b) prosthetischen Gruppen;
c) fluoreszierenden Materialien;
d) luminiszierenden Materialien;
e) bioluminiszierenden Materialien; und
f) radioaktiven Materialien.

14. Der Antikörper oder immunologisch aktive Teil davon gemäß Anspruch 11, wobei der Antikörper ausgewählt ist aus der Gruppe bestehend aus:
a) einem monoklonalen Antikörper;
b) einem polyklonalen Antikörper;
c) einem Fab Fragment;
d) einem F(ab')₂ Fragment;
e) einem chimeren Antikörper;
f) einem humanisierten Antikörper; und
g) einem humanen Antikörper.

15. Verfahren zur Herstellung eines Polypeptids ausgewählt aus der Gruppe bestehend aus:
a) einem Polypeptid umfassend die Aminosäuresequenz nach SEQ ID NO: 2; und
b) einem Polypeptid umfassend die Aminosäuresequenz nach SEQ ID NO: 2, wobei das Fragment zumindest 1200 zusammenhängende Aminosäuresequenzen aus SEQ ID NO: 2 umfasst;
wobei das Verfahren das Kultivieren einer Wirtszelle nach Anspruch 5 unter Bedingungen umfasst, unter denen das Nukleinsäuremolekül exprimiert wird.

16. Verfahren zur Detektion der Gegenwart eines Polypeptids nach Anspruch 8 in einer Probe, das Verfahren umfassend:
a) Kontaktieren der Probe mit einer Verbindung, die selektiv an ein Polypeptid nach Anspruch 8 bindet; und
b) Bestimmung ob die Verbindung an das Polypeptid in der Probe bindet.

17. Das Verfahren nach Anspruch 16, wobei die Verbindung, die an das Polypeptid bindet, ein Antikörper ist.

18. Kit umfassend einen Antikörper der selektiv an ein Polypeptid nach Anspruch 8 bindet und eine Gebrauchsanleitung.

19. Verfahren zur Detektion der Gegenwart eines Nukleinsäuremoleküls nach Anspruch 1 in einer Probe umfassend die Schritte:
a) Kontaktieren der Probe mit einer Nukleinsäuresonde oder einem Primer, die / der selektiv mit dem Nukleinsäuremolekül hybridisiert; und
b) Bestimmung ob die Nukleinsäuresonde oder der Primer an das Nukleinsäuremolekül in der Probe bindet.

20. Das Verfahren nach Anspruch 19, wobei die Probe mRNA Moleküle umfasst und mit der Nukleinsäuresonde in Kontakt gebracht wird.

21. Kit umfassend eine Nukleinsäuresonde oder einen Primer, die / der selektiv an ein Nukleinsäuremolekül nach Anspruch 1 bindet, sowie eine Gebrauchsanleitung.

22. Verfahren zur Identifizierung einer Verbindung, die an eine Polypeptid nach Anspruch 8 bindet, umfassend die Schritte:
a) Kontaktieren eines Polypeptids oder einer Zelle, die ein Polypeptid nach Anspruch 8 exprimiert, mit einer Testverbindung; und
b) Bestimmen ob das Polypeptid an die Testverbindung bindet.

23. Verfahren nach Anspruch 22, wobei die Bindung der Testverbindung an das Polypeptid mittels eines Verfahrens detektiert wird, das ausgewählt ist aus der Gruppe bestehend aus:
a) Detektion der Bindung mittels direkter Detektion der Bindung der Testverbindung und des Polypeptids; und
b) Detektion der Bindung unter Verwendung eines kompetitiven Bindungsassays.

24. Verfahren zum Modulieren der Aktivität eines Polypeptids nach Anspruch 8, umfassend das Kontaktieren eines Polypeptids oder einer Zelle, die ein Polypeptid nach Anspruch 8 exprimiert, mit einer ausreichenden Konzentration eines Antikörpers, der an das Polypeptid bindet, um die Aktivität des Polypeptids zu modulieren.

25. Verfahren zur Identifikation eines Wirkstoffs, der die Aktivität eines Polypeptids nach Anpruch 8 moduliert, umfassend:
a) Kontaktieren eines Polypeptids nach Anspruch 8 mit einem Test-Wirkstoff; und
b) Bestimmung des Effekts der Testverbindung auf die Aktivität des Polypeptids um damit einen Wirkstoff zu identifizieren, der die Aktivität des Polypeptids moduliert.

26. Verwendung eines Wirkstoffs, der die Aktivität oder Expression einer Nukleinsäure nach Anspruch 1 oder eines Polypeptids nach Anspruch 8 moduliert zur Herstellung eines Medikaments zur Behandlung oder Prävention einer zellulären proliferativen oder differentiativen Störung in einem Subjekt, die durch eine abnormale Aktivität oder Expression einer Nukleinsäure nach Anspruch 1 oder eines Polypeptids nach Anspruch 8 **gekennzeichnet** ist, wobei die Behandlung die Verabreichung einer wirksamen Menge des Wirkstoffs, der die Aktivität oder die Expression einer Nukleinsäure nach Anspruch 1 oder eines Polypeptids nach Anspruch 8 moduliert, an das Subjekt umfasst, sodass die zelluläre proliferative oder differentiative Störung sich bessert oder verhindert wird, wobei der Wirkstoff ausgewählt ist aus der Gruppe bestehend aus einem Polypeptid nach Anspruch 8, einem Nukleinsäuremolekül nach Anspruch 1, einem Antikörper, einem Ribozym und einem Antisense-Molekül.

27. Die Verwendung nach Anspruch 26, wobei die zelluläre proliferative oder differentiative Störung Lungenkrebs, Darmkrebs oder Brustkrebs ist.

28. Ein in vitro Verfahren zur Identifizierung eines Wirkstoffs, der die Aktivität oder die Expression eines Polypeptids nach Anspruch 8 oder eines Nukleinsäuremoleküls nach Anspruch 1 moduliert, das Verfahren umfassend:
a) Kontaktieren des Polypeptids oder der Nukleinsäure mit einem Wirkstoff; und
b) Bestimmung des Effekts des Wirkstoffs auf die Aktivität oder die Expression des Polypeptids oder der Nukleinsäure; wobei der Wirkstoff ausgewählt ist aus der Gruppe bestehend aus einem Polypeptid nach Anspruch 8, einem Nukleinsäuremolekül nach Anspruch 1, einem Antikörper, einem Ribozym und einem Antisense-Molekül.

29. Das Verfahren nach Anspruch 28, das das Kontaktieren eines Polypeptids nach Anspruch 8 mit dem Wirkstoff umfasst und die Bestimmung des Effekts des Wirkstoffs auf die Fähigkeit des Polypeptids die Hydrolyse von Phosphatidylinositol zu katalysieren, oder welches das Kontaktieren eines Polypeptids nach Anspruch 8 mit dem Wirkstoff umfasst und die Bestimmung des Effekts des Wirkstoffs auf die Fähigkeit des Polypeptids mit dem Ras Protein zu assoziieren, oder welches das Kontaktieren eines Polypeptids nach Anspruch 8 mit dem Wirkstoff umfasst und die Bestimmung des Effekts des Wirkstoffs auf die Fähigkeit eine Guaninnukleotid Austauschaktivität zu vermitteln.

30. In vitro Verfahren zum Modulieren der Aktivität einer Zelle, die ein Polypeptid nach Anspruch 8 oder ein Nukleinzsäuremolekül nach Anspruch 1 exprimiert, umfassend das Kontaktieren der Zelle mit einer Menge eines Wirkstoffs, die die Aktivität oder Expression einer Nukleinsäure nach Anspruch 1 oder eines Polypeptids nach Anspruch 8 moduliert, sodass die Aktivität der Zelle moduliert wird; wobei der Wirkstoff ausgewählt ist aus der Gruppe bestehend aus einem Polypeptid nach Anspruch 8, einem Nukleinsäuremolekül nach Anspruch 1, einem Antikörper, einem Ribozym und einem Antisense-Molekül.

## Revendications

1. Molécule d'acide nucléique isolée choisie dans le groupe constitué par :
a) une molécule d'acide nucléique consistant en une séquence nucléotidique qui est identique à au moins 80 % à la longueur totale de la séquence nucléotidique de SEQ ID NO:1 ou qui est identique à au moins 92 % à la longueur totale de la séquence nucléotidique de SEQ ID NO:3 ;
b) une molécule d'acide nucléique qui code pour un polypeptide comprenant la séquence d'acides aminés de SEQ ID NO:2 ; et
c) une molécule d'acide nucléique qui code pour un fragment d'un polypeptide comprenant la séquence d'acides aminés de SEQ ID NO:2, le fragment comprenant au moins 1 200 acides aminés contigus de SEQ ID NO:2.

2. Molécule d'acide nucléique isolée selon la revendication 1, qui est choisie dans le groupe constitué par :
a) un acide nucléique comprenant la séquence nucléotidique de SEQ ID NO:1, SEQ ID NO:3 ; et
b) une molécule d'acide nucléique qui code pour un polypeptide comprenant la séquence d'acides aminés de SEQ ID NO:2.

3. Molécule d'acide nucléique selon la revendication 1, comprenant en outre des séquences d'acide nucléique vecteurs.

4. Molécule d'acide nucléique selon la revendication 1, comprenant en outre des séquences d'acide nucléique codant pour un polypeptide hétérologue.

5. Cellule hôte qui contient la molécule d'acide nucléique selon la revendication 1.

6. Cellule hôte selon la revendication 5, qui est une cellule hôte de mammifère.

7. Cellule hôte de mammifère non humain contenant la molécule d'acide nucléique selon la revendication 1.

8. Polypeptide isolé choisi dans le groupe constitué par :
a) un polypeptide qui est codé par une molécule d'acide nucléique consistant en une séquence nucléotidique qui est identique à au moins 80 % à la longueur totale de la séquence nucléotidique de SEQ ID NO:1 ou qui est identique à au moins 92 % à la longueur totale de la séquence nucléotidique de SEQ ID NO:3 ; et
b) un fragment d'un polypeptide comprenant la séquence d'acides aminés de SEQ ID NO:2, le fragment comprenant au moins 1 200 acides aminés contigus de SEQ ID NO:2.

9. Polypeptide isolé selon la revendication 8, comprenant la séquence d'acides aminés de SEQ ID NO:2.

10. Polypeptide selon la revendication 8, comprenant en outre des séquences d'acides aminés hétérologues.

11. Anticorps ou partie immunologiquement active de celui-ci, qui se lie de façon sélective à un polypeptide choisi dans le groupe constitué par :
a) un polypeptide qui est codé par une molécule d'acide nucléique consistant en une séquence nucléotidique qui est identique à au moins 80 % à la longueur totale de la séquence nucléotidique de SEQ ID NO:1 ou qui est identique à au moins 92 % à la longueur totale de la séquence nucléotidique de SEQ ID NO:3 ; et
b) un fragment d'un polypeptide consistant en la séquence d'acides aminés de SEQ ID NO:2, le fragment comprenant au moins 1 200 acides aminés contigus de SEQ ID NO:2.

12. Anticorps ou partie immunologiquement active de celui-ci selon la revendication 11, dans lequel l'anticorps est marqué de façon détectable.

13. Anticorps ou partie immunologiquement active de celui-ci selon la revendication 12, dans lequel le marqueur détectable est choisi dans le groupe constitué par :
a) des enzymes ;
b) des groupes prosthétiques ;
c) des matières fluorescentes ;
d) des matières luminescentes ;
e) des matières bioluminescentes ; et
f) des matières radioactives.

14. Anticorps ou partie immunologiquement active de celui-ci selon la revendication 11, dans lequel l'anticorps est choisi dans le groupe constitué par :
a) un anticorps monoclonal ;
b) un anticorps polyclonal ;
c) un fragment Fab ;
d) un fragment F(ab')₂ ;
e) un anticorps chimérique ;
f) un anticorps humanisé ; et
g) un anticorps humain.

15. Procédé de production d'un polypeptide choisi dans le groupe constitué par :
a) un polypeptide comprenant la séquence d'acides aminés de SEQ ID NO:2 ; et
b) un polypeptide comprenant un fragment de la séquence d'acides aminés de SEQ ID NO:2, le fragment comprenant au moins 1 200 acides aminés contigus de SEQ ID NO:2 ;
le procédé comprenant la culture de la cellule hôte selon la revendication 5 dans des conditions dans lesquelles la molécule d'acide nucléique est exprimée.

16. Procédé de détection de la présence d'un polypeptide selon la revendication 8 dans un échantillon, comprenant les opérations consistant à :
a) mettre en contact l'échantillon avec un composé qui se lie de façon sélective à un polypeptide selon la revendication 8 ; et
b) déterminer si le composé se lie ou non au polypeptide dans l'échantillon.

17. Procédé selon la revendication 16, dans lequel le composé qui se lie au polypeptide est un anticorps.

18. Coffret comprenant un anticorps qui se lie de façon sélective à un polypeptide selon la revendication 8, et instructions pour l'utilisation.

19. Procédé de détection de la présence d'une molécule d'acide nucléique selon la revendication 1 dans un échantillon, comprenant les étapes consistant à :
a) mettre en contact l'échantillon avec une sonde ou amorce d'acide nucléique qui s'hybride de façon sélective à la molécule d'acide nucléique ; et
b) déterminer si la sonde ou amorce d'acide nucléique se lie ou non à une molécule d'acide nucléique dans l'échantillon.

20. Procédé selon la revendication 19, dans lequel l'échantillon comprend des molécules d'ARNm et est mis en contact avec une sonde d'acide nucléique.

21. Coffret comprenant une sonde ou amorce d'acide nucléique qui s'hybride de façon sélective à une molécule d'acide nucléique selon la revendication 1, et instructions pour l'utilisation.

22. Procédé d'identification d'un composé qui se lie à un polypeptide selon la revendication 8, comprenant les étapes consistant à :
a) mettre en contact un polypeptide, ou une cellule exprimant un polypeptide selon la revendication 8 avec un composé de test ; et
b) déterminer si le polypeptide se lie ou non au composé de test.

23. Procédé selon la revendication 22, dans lequel la liaison du composé de test au polypeptide est détectée par un procédé choisi dans le groupe constitué par :
a) la détection de la liaison par détection directe de la liaison composé de test/polypeptide ; et
b) la détection de la liaison à l'aide d'un essai de liaison par compétition.

24. Procédé pour moduler l'activité d'un polypeptide selon la revendication 8, comprenant la mise en contact d'un polypeptide ou d'une cellule exprimant un polypeptide selon la revendication 8 avec un anticorps qui se lie au polypeptide dans une concentration suffisante pour moduler l'activité du polypeptide.

25. Procédé pour identifier un agent qui module l'activité d'un polypeptide selon la revendication 8, comprenant les opérations consistant à :
a) mettre en contact un polypeptide selon la revendication 8 avec un agent de test ; et
b) déterminer l'effet du composé de test sur l'activité du polypeptide pour identifier de cette façon un agent qui module l'activité du polypeptide.

26. Utilisation d'un agent qui module l'activité ou l'expression d'un acide nucléique selon la revendication 1 ou d'un polypeptide selon la revendication 8 dans la fabrication d'un médicament pour le traitement ou la prévention d'un trouble de la prolifération ou de la différenciation cellulaire **caractérisé par** une activité ou une expression aberrante d'un acide nucléique selon la revendication 1 ou d'un polypeptide selon la revendication 8 dans un sujet, le traitement comprenant l'administration au sujet d'une quantité efficace de l'agent qui module l'activité ou l'expression d'un acide nucléique selon la revendication 1 ou d'un polypeptide selon la revendication 8, de telle sorte que le trouble de la prolifération ou de la différenciation cellulaire est amélioré ou empêché, l'agent étant choisi dans le groupe constitué par un polypeptide selon la revendication 8, une molécule d'acide nucléique selon la revendication 1, un anticorps, un ribozyme et une molécule antisens.

27. Utilisation selon la revendication 26, dans laquelle le trouble de la prolifération ou de la différenciation cellulaire est le cancer du poumon, le cancer du côlon ou le cancer du sein.

28. Procédé in vitro pour identifier un agent qui module l'activité ou l'expression d'un polypeptide selon la revendication 8 ou d'une molécule d'acide nucléique selon la revendication 1, le procédé comprenant les opérations consistant à :
a) mettre en contact le polypeptide ou l'acide nucléique avec un agent ; et
b) déterminer l'effet de l'agent sur l'activité ou l'expression du polypeptide ou de l'acide nucléique, l'agent étant choisi dans le groupe constitué par un polypeptide selon la revendication 8, une molécule d'acide nucléique selon la revendication 1, un anticorps, un ribozyme et une molécule antisens.

29. Procédé selon la revendication 28, qui comprend la mise en contact d'un polypeptide selon la revendication 8 avec l'agent et la détermination de l'effet de l'agent sur l'aptitude du polypeptide à catalyser l'hydrolyse du phosphatidylinositol, ou qui comprend la mise en contact d'un polypeptide selon la revendication 8 avec l'agent et la détermination de l'effet de l'agent sur l'aptitude du polypeptide à s'associer à une protéine Ras, ou qui comprend la mise en contact d'un polypeptide selon la revendication 8 avec l'agent et la détermination de l'effet de l'agent sur l'aptitude du polypeptide à assurer la méditation de l'activité d'échange du nucléotide à guanine.

30. Procédé in vitro de modulation de l'activité d'une cellule exprimant un polypeptide selon la revendication 8 ou une molécule d'acide nucléique selon la revendication 1, comprenant la mise en contact de la cellule avec une quantité d'un agent qui module l'activité ou l'expression d'un acide nucléique selon la revendication 1 ou d'un polypeptide selon la revendication 8, de telle sorte que l'activité de la cellule est modulée ; l'agent étant choisi dans le groupe constitué par un polypeptide selon la revendication 8, une molécule d'acide nucléique selon la revendication 1, un anticorps, un ribozyme et une molécule antisens.
